(19) Europäisches Patentamt
European Patent Office
Office européen des brevets

(11) EP 0 563 715 B1

(12) **EUROPÄISCHE PATENTSCHRIFT**

(45) Veröffentlichungstag und Bekanntmachung des Hinweises auf die Patenterteilung: **14.08.1996 Patentblatt 1996/33**

(21) Anmeldenummer: **93104595.9**

(22) Anmeldetag: **20.03.1993**

(51) Int. Cl.$^6$: **C07C 259/06**, C07C 275/64, C07D 333/20, C07D 213/42, A61K 31/16, A61K 31/17, C07D 521/00

(54) **Oxyalkine, diese enthaltende Arzneimittel sowie ein Verfahren zur Herstellung dieser Verbindungen und Arzneimittel**

Oxyalkynes, pharmaceutical compositions containing same as well as a process for the preparation of these compounds and pharmaceutical compositions

Oxyalkynes, compositions pharmaceutiques qui leurs contiennent et un procédé pour la préparation de ces composés et ces compositions pharmaceutiques

(84) Benannte Vertragsstaaten:
**AT BE CH DE DK ES FR GB GR IE IT LI LU MC NL PT SE**

(30) Priorität: **30.03.1992 DE 4210322**

(43) Veröffentlichungstag der Anmeldung: **06.10.1993 Patentblatt 1993/40**

(73) Patentinhaber: **Grünenthal GmbH**
**D-52078 Aachen (DE)**

(72) Erfinder:
- **Seipp, Ulrich, Dr.**
**W-5100 Aachen (DE)**
- **Vollenberg, Werner, Dr.**
**W-5190 Stolberg (DE)**
- **Englberger, Werner, Dr.**
**W-5190 Stolberg (DE)**
- **Geist, Cornelia, Dr.**
**W-5100 Aachen (DE)**
- **Haurand, Michael, Dr.**
**W-5190 Stolberg (DE)**

(56) Entgegenhaltungen:
**EP-A- 0 279 281**
**EP-A- 0 292 699**
**EP-A- 0 452 908**
**EP-A- 0 468 281**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 563 715 B1

Printed by Rank Xerox (UK) Business Services
2.13.0/3.4

## Beschreibung

Die Erfindung betrifft neue Oxyalkine der allgemeinen Formel I

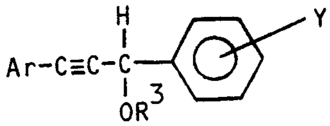

solche Verbindungen enthaltende Arzneimittel sowie ein Verfahren zur Herstellung dieser Verbindungen und Arzneimittel.

Polyungesättigte höhere Fettsäuren, beispielsweise Arachidonsäure, dienen im Stoffwechsel des Menschen und von Säugetieren als Substrate für die enzymatisch katalysierte Bildung physiologisch und pathophysiologisch bedeutsamer Eicosanoide, wie Prostaglandine und Leukotriene. Dabei wird die Bildung von Prostaglandinen durch das Enzym Cyclooxygenase und die Leukotrienbildung durch das Enzym 5-Lipoxygenase eingeleitet.

Während die Prostaglandine eine Anzahl erwünschter Wirkungen im Organismus entfalten, ist von Leukotrienen bekannt, daß sie eine wichtige Rolle bei der Entstehung von lebensbedrohlichen Situationen, beispielsweise beim anaphylaktischen und septischen Schock, bei allergischen Reaktionen, Bronchokonstriktionen und Asthma spielen. Aufgrund der Vielzahl der mit Leukotrienen in Zusammenhang stehenden unerwünschten Effekte hat es in der Vergangenheit nicht an Bemühungen gefehlt, chemisch und metabolisch stabile Substanzen zur Verfügung zu stellen, die die Prostaglandinbildung im Organismus unbeeinflußt lassn, gleichzeitig aber möglichst selekiv Lipoxygenase hemmen und so die Bildung der unerwünschten Leukotriene verhindern. Beispielsweise sind Acetohydroxamsäureverbindungen, die die 5-Lipoxygenase ausgeprägt, Cyclooxygenase aber nur wenig hemmen, aus einer Arbeit von Tateson et al. in Brit. J. Pharmacol. 94, 528 (1988) bekannt.

Die aus EP 468 281 bekannten substituierten Phenylacetylene haben ebenfalls eine spezifische Hemmwirkung gegenüber 5-Lipoxygenase. Diese Verbindungen entsprechen der Formel

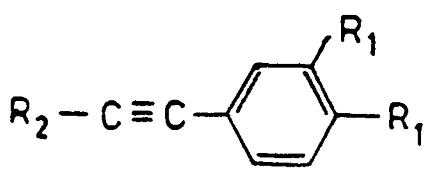

in der einer der beiden Reste $R_1$ H und der andere -$CHR_3$-N(OH)-CO-$R_4$ ist, $R_3$ H, $CH_3$ oder $CH_2CH_3$ und $R_4$ $CH_3$ oder $NH_2$ bedeutet und $R_2$ einen substituierten oder unsubstituierten Phenyl-, Naphthyl, Thienyl, Benzothienyl, Pyridyl oder Benzopyridylrest darstellt.

Es wurde nun gefunden, daß bestimmte toxikologisch unbedenkliche, chemisch und metabolisch stabile Oxyalkine ohne Beeinflussung der Cyclooxygenaseaktivität entweder 5-Lipoxygenase oder 5- und 12-Lipoxygenase hemmen.

Gegenstand der Erfindung sind dementsprechend Oxyalkine der allgemeinen Formel I

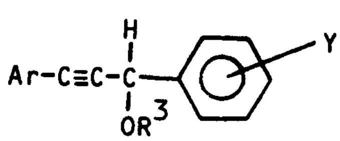

in der der meta- oder para-ständige Substituent Y

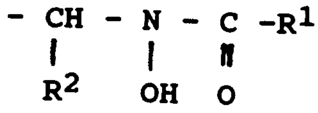

$R^1$ $CH_3$ oder $NH_2$, $R^2$ H oder $CH_3$ und $R^3$ H, $C_{1-3}$-Alkyl oder $COCH_3$ bedeuten und Ar einen aromatischen Rest aus der Gruppe

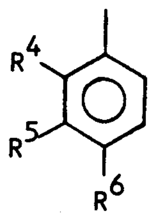 , 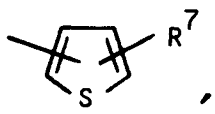 , 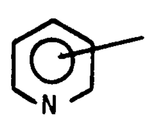 und 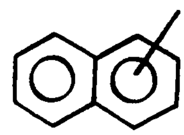

darstellt, mit der Maßgabe, daß die Substituenten $R^4$, $R^5$ und $R^6$ gleich oder verschieden sind und jeweils H, F, Cl, Br, $C_{1-6}$-Alkyl, $CF_3$ oder $C_{1-6}$-Alkoxy bedeuten und $R^7$ H, F, Cl, Br, $C_{1-3}$-Alkyl oder $CF_3$ ist,
als Racemate oder Diastereomerengemische oder in optisch aktiver Form.

Sofern der aromatische Rest Ar den Pyridylrest bedeutet, handelt es sich um den 2-, 3- oder 4-Pyridylrest, bedeutet Ar den Naphthylrest, handelt es sich um den 1- oder 2-Naphthylrest.

Verbindungen der allgemeinen Formel I, in denen $R^2$ H bedeutet und insbesondere solche Verbindungen, in denen $R^3$ H bedeutet, werden bevorzugt. Sofern Ar ein Phenylrest ist, können die Substituenten $R^4$, $R^5$ und $R^6$ gleich oder verschieden sein. Vorzugsweise stellt jeder dieser Substituenten H, F, Cl oder $OCH_3$ dar. Oxyalkine, in denen Ar ein Phenylrest ist, in dem wenigstens einer der Substituenten $R^4$, $R^5$ und $R^6$ das Wasserstoffatom bedeutet, werden bevorzugt. Besonders bevorzugt werden Oxyalkine, in denen Ar den unsubstituierten Phenylrest oder den 4-Fluorphenylrest darstellt. Sofern Ar den 2- oder 3-Thienylrest bedeutet, ist $R^7$ vorzugsweise Wasserstoff. N-Hydroxy-N-[4-[1-hydroxy-3-phenyl-prop-2-in-1-yl]benzyl]acetamid und N-Hydroxy-N-[4-[1-hydroxy-3-(4-fluorphenyl)prop-2-in-1-yl]benzyl]harnstoff sind Beispiele besonders geeigneter Verbindungen.

Die erfindungsgemäßen Oxyalkine hemmen selektiv 5-Lipoxygenase oder selektiv 5- und 12-Lipoxygenase, während die Aktivität der Cyclooxygenase bei gleicher Konzentration nicht beeinflußt wird. Die daraus resultierende Hemmung der Bildung der pharmakologisch äußerst wirksamen Arachidonsäuremetabolite 5-Hydroxyeicosatretraensäure (5-HETE), 12-HETE, 5S,12S-Di-HETE sowie der Leukotriene, beispielsweise $LTB_4$, $LTC_4$, $LTD_4$ und $LTE_4$, verleiht den erfindungsgemäßen Verbindungen zahlreiche physiologisch wertvolle Eigenschaften, beispielsweise antianaphylaktische, antiasthmatische, antiallergische, antiphlogistische, blutdrucksenkende, durchblutungsfördernde (coronarer und cerebraler Kreislauf) sowie antipsoriatische Wirkungen. Des weiteren vermindern die erfindungsgemäßen Verbindungen die Leukozytenaggregation sowie die Bildung von Leukozytenthromben. Da die erfindungsgemäßen Verbindungen der allgemeinen Formel I chemisch und metabolisch für eine therapeutische Anwendung stabil und lagerfähig sind, eignen sie sich zum Einsatz als Arzneimittel, beispielsweise als Antiallergikum, Antianaphylaktikum, Antiphlogistikum, Antiasthmatikum, Antihypertensivum, als antithrombotisches Mittel, als Mittel zur Prophylaxe oder Therapie von ischämischem Herzinfarkt, als Mittel zur Behandlung von Störungen coronarer und/oder cerebraler Gefäße und/oder als Mittel zur Hemmung der Metastasenbildung.

Oxyalkine der allgemeinen Formel I sind toxikologisch unbedenkiich, so daß sie als solche in geeigneten pharmazeutischen Zubereitungen an Mensch und Tier verabreicht werden können.

Weiterer Erfindungsgegenstand sind dementsprechend Arzneimittel, die als Wirkstoff wenigstens ein Oxyalkin der allgemeinen Formel I enthalten.

Die an den Patienten zu verabreichende Wirkstoffmenge variiert in Abhängigkeit vom Gewicht des Patienten, von der Applikationsart, der Indikation und dem Schweregrad der Erkrankung. Unter Berücksichtigung dieser Faktoren liegt der Wirkstoffgehalt pro Einzeldosis im allgemeinen zwischen 0,01 und 1000 mg, wobei Zubereitungsformen für die orale Applikation 1 bis 1000 mg Wirkstoff, Zubereitungsformen für die parenterale Applikation 0,1 bis 1000 mg Wirkstoff und Zubereitungsformen für die topische und inhalative Anwendung 0,01 bis 100 mg Wirkstoff enthalten können.

Für viele prophylaktische oder therapeutische Anwendungen der erfindungsgemäßen Verbindungen eignen sich topisch und insbesondere oral anwendbare Zubereitungsformen, wie Tabletten, Dragees, Kapseln, Granulate, Salben, Tropfen, Säfte und Sirupe, aber auch Suppositorien sowie perkutane Applikationszubereitungen, beispielsweise Wirkstoffe in einem Depot in gelöster Form oder Pflaster, die gegebenenfalls einen Zusatz von die Hautpenetration fördernden Mitteln enthalten. Vorteilhaft werden oral, rektal oder perkutan anwendbaren Zubereitungsformen so hergestellt,

daß daraus der Wirkstoff verzögert freigesetzt wird, um so über einen längeren Zeitraum, beispielsweise von 24 Stunden, eine gleichmäßige Versorgung des Patienten mit dem Wirkstoff zu gewährleisten.

Für die parenterale Applikation eignen sich darüber hinaus Arzneimittel in Form von Lösungen, Suspensionen oder leicht rekonstituierbaren Trockenzubereitungen. Sprays eignen sich für die intranasale oder inhalative Applikation.

Alle vorstehend aufgeführten pharmazeutischen Zubereitungsformen sind an sich bekannt, und da Oxyalkine der allgemeinen Formel I chemisch stabil sind, wirft ihre Einarbeitung in diese Zubereitungsformen für den Fachmann keinerlei Probleme auf. Bei der erfindungsgemäßen Herstellung dieser Arzneimittel muß selbstverständlich mit der üblichen Sorgfalt bei Auswahl der Hilfsstoffe, beispielsweise Trägermaterialien, Lösungsmittel, Verdünnungsmittel, Farbstoffe, Geschmackskorrigenzien, Bindemittel und Tablettensprengstoffe, vorgegangen werden, und insbesondere bei der Herstellung von parenteral anzuwendenden Zubereitungsformen ist auf Sterilität und - sofern diese in wäßriger Form vorliegen - Isotonie zu achten.

Weiterer Erfindungsgegenstand ist ein Verfahren zur Herstellung eines Oxyalkins der allgemeinen Formel I

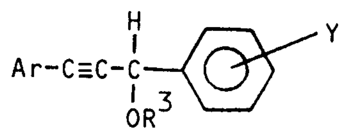

in der der meta- oder para-ständige Substituent Y

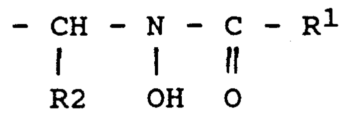

$R^1$ $CH_3$ oder $NH_2$, $R^2$ H oder $CH_3$ und $R^3$ H, $C_{1-3}$-Alkyl oder $COCH_3$ bedeuten und Ar einen aromatischen Rest aus der Gruppe

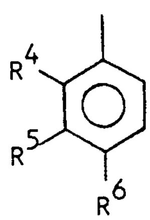 , 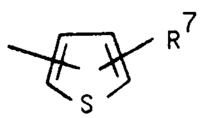 , 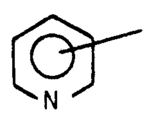 und 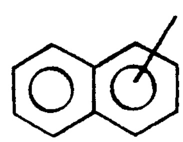

darstellt, mit der Maßgabe, daß die Substituenten $R^4$, $R^5$ und $R^6$ gleich oder verschieden sind und jeweils H, F, Cl, Br, $C_{1-6}$-Alkyl, $CF_3$ oder $C_{1-6}$-Alkoxy bedeuten und $R^7$ H, F, Cl, Br, $C_{1-3}$-Alkyl oder $CF_3$ ist, welches dadurch gekennzeichnet ist, daß man ein Alkin der allgemeinen Formel II

$$Ar - C \equiv CH$$

in Gegenwart einer Base mit einem partiell geschützten Terephthal- oder Isophthalaldehyd der allgemeinen Formel III

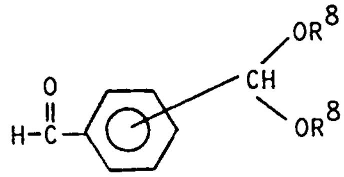

in der $R^8$ $CH_3$ oder $CH_2CH_3$ bedeutet oder die beiden $R^8$-Reste zusammen die Ethylengruppe darstellen, zu einem Acetal der allgemeinen Formel IV

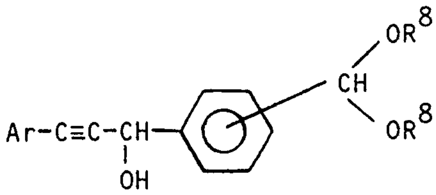

umsetzt, das Acetal mit einer Säure in einem wasserhaltigen Lösungsmittel in einen Hydroxyaldehyd der allgemeinen Formel V

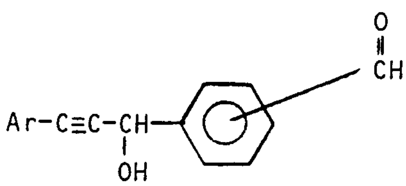

überführt, anschließend aus dem Hydroxyaldehyd ein Oxim der allgemeinen Formel VI

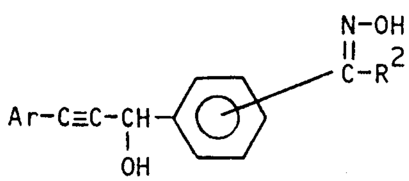

herstellt, indem man entweder
einen Hydroxyaldehyd der allgemeinen Formel V direkt mit Hydroxylamin oder einem seiner Salze in Gegenwart einer Base in ein Oxim der allgemeinen Formel VI überführt
oder
das alkoholische Proton des Hydroxyaldehyds der allgemeinen Formel V durch eine gegenüber Grignardreagenzien inerte, durch Säuren abspaltbare Gruppe ersetzt, anschließend mit einem Methylmagnesiumhalogenid in einem wasserfreien nukleophilen Lösungsmittel umsetzt, den erhaltenen sekundären Alkohol mit einem Oxydationsmittel zu einer Ketoverbindung oxydiert und diese durch Abspaltung der gegenüber Grignardreagenzien inerten Gruppe mit Säuren in eine Ketoverbindung der allgemeinen Formel Va

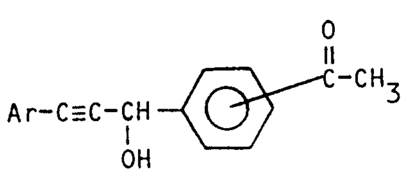

überführt und aus dieser durch Umsetzung mit Hydroxylamin oder einem seiner Salze in Gegenwart einer Base ein Oxim der allgemeinen Formel VI herstellt,
anschließend das erhaltene Oxim mit einem borhaltigen Reduktionsmittel in Gegenwart einer Säure und gegebenenfalls eines $C_{1-3}$-Alkylalkohols zum Hydroxylamin der allgemeinen Formel VII

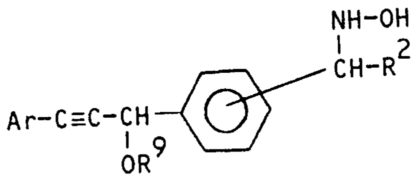

in der $R^9$ H oder $C_{1-3}$-Alkyl bedeutet,
reduziert und das erhaltene Hydroxylamin entweder

A. mit Trimethylsilylisocyanat oder Alkalicyanat und anschließender Hydrolyse oder mit Phosgen und anschließender Aminolyse

oder

B. mit einem Acetylierungsmittel und anschließender selektiver basischer Hydrolyse oder Alkoholyse der O-Acetylgruppe am Stickstoffatom und gegebenenfalls Umwandlung der propargylischen $OCOCH_3$-Gruppe in die OH-Gruppe

in ein Oxyalkin der allgemeinen Formel I überführt.

Acetale der allgemeinen Formel IV lassen sich in bekannter Weise herstellen, indem ein Alkin der allgemeinen Formel II bei -75° C bis +25° C in einem wasserfreien Lösungsmittel oder Lösungsmittelgemisch, beispielsweise Tetrahydrofuran, Diethylether, Ethylen- und/oder Diethylenglykoldimethylether, zuerst mit einer starken Base, wie n-Butyllithium oder einem Grignardreagenz, und anschließend mit, bezogen auf die eingesetzte Menge an Alkin, 1 bis 1,1 Mol eines partiell geschützten Terephthal- oder Isophthalaldehydes, beispielsweise Terephthalaldehydmonodimethylacetal oder -monodiethylacetal oder Isophthalaldehydmonodimethylacetal oder -monodiethylacetal, umgesetzt wird. Durch Umsetzung der erhaltenen Acetale mit einer Säure, beispielsweise Salzsäure oder dem Pyridiniumsalz der p-Toluolsulfonsäure, in einem wasserhaltigen Lösungsmittel oder Lösungsmittelgemisch, wie Methanol, Ethanol und/oder Tetrahydrofuran, bei 0° C bis 50° C sind die Hydroxyaldehyde der allgemeinen Formel V zugänglich.

Zur Herstellung von Oxyalkinen der allgemeinen Formel I, in denen der Rest $R^2$ $CH_3$ bedeutet, wird zunächst das alkoholische Proton eines Hydroxyaldehyds der allgemeinen Formel V durch eine gegenüber Grignardreagenzien inerte, durch Säuren abspaltbare Gruppe, beispielsweise durch die Tetrahydropyranyl-, tert.-Butyldiphenylsilyl-, tert.-Butyldimethylsilyl-, Isopropyldimethylsilyl-, Triethylsilyl- oder Trimethylsilylgruppe, in bekannter Weise ersetzt und anschließend der Aldehyd mit Methylmagnesiumjodid, -bromid oder -chlorid in einem wasserfreien, nukleophilen Lösungsmittel oder Lösungsmittelgemisch, das kein aktives Wasserstoffatom enthält, beispielsweise Tetrahydrofuran, Ethylenglykoldimethylether und/oder Diethylether, bei Temperaturen zwischen 0° C und 50° C in den entsprechenden sekundären Alkohol überführt. Durch Oxydation des sekundären Alkohols mit beispielsweise $SO_3$-Pyridinkomplex in Dimethylsulfoxid (J. Am. Chem. Soc. 89, 5505 (1967)), Braunstein (J. Org. Chem. 26, 2973 (1961)), Pyridiniumdichromat (Tetrahedron Lett. 1979, 399), Pyridiniumchlorochromat (Tetrahedron Lett. 1975, 2647), Dicyclohexylcarbodiimid/Dimethylsulfoxid/Pyridiniumtrifluoracetat (J. Am. Chem. Soc. 87, 5661 (1965)) oder Oxalylchlorid/Dimethylsulfoxid (J. Org. Chem. 43, 2480 (1978)), und anschließende Abspaltung der gegenüber Grignardreagenzien inerten Gruppe mit einer Säure, beispielsweise Salzsäure oder p-Toluolsulfonsäure, Pyridiniumsalz, in einem alkoholfreien, wasserhaltigen Lösungsmittel oder Lösungsmittelgemisch, beispielsweise Tetrahydrofuran, Diethylether und/oder Essigsäureethylester, bei Temperaturen zwischen 20° C und 70° C läßt sich eine Ketoverbindung der allgemeinen Formel Va

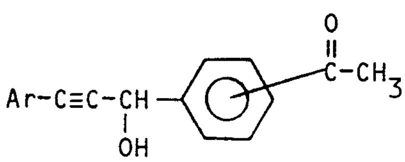

herstellen. Aus dieser Ketoverbindung kann ein Oxim der allgemeinen Formel VI in bekannter Weise durch Umsetzung mit 1,2 bis 2 Mol Hydroxylamin und/oder einem seiner Salze, beispielsweise Hydroxylammoniumchlorid, in einem polaren Lösungsmittel oder Lösungsmittelgemisch, beispielsweise Methanol, Ethanol, Tetrahydrofuran oder wäßrigen Mischungen dieser Lösungsmittel, in Gegenwart einer Base, beispielsweise Pyridin, Natriumacetat, Kalium- oder Natriumcarbonat, bei Temperaturen zwischen 15° C und 60° C erhalten werden.

Zur Herstellung der bevorzugten Oxyalkine der allgemeinen Formel I, in denen der Rest $R^2$ H bedeutet, wird ein Hydroxyaldehyd der allgemeinen Formel V direkt mit Hydroxylamin und/oder einem seiner Salze unter den oben genannten Bedingungen in ein Oxim der allgemeinen Formel VI überführt.

Oxime der allgemeinen Formel VI werden vorzugsweise mit einem Borhydrid oder einem Boran-Amin-Komplex, beispielsweise Natriumcyanoborhydrid in Essigsäure oder methanolischer, ethanolischer, propanolischer und/oder isopropanolischer Salzsäure bei Temperaturen zwischen 20 und 60° C oder Boran-Pyridin-Komplex in einem oder mehreren Lösungsmitteln wie Tetrahydrofuran, Methanol und/oder Ethanol in Gegenwart von Salzsäure bei Temperaturen zwischen etwa 0 und 20° C, zu den Hydroxylaminen der allgemeinen Formel VII reduziert (J. B. Summers et al., J. Med. Chem. 31, 1960 (1988)). Während der Reduktion in saurem alkoholischem Milieu wird gleichzeitig die propargylische Hydroxygruppe verethert. Vorzugsweise wird jedoch die Reduktion in Abwesenheit von $C_{1-3}$-Alkylalkoholen durchgeführt.

Zur Herstellung von Oxyalkinen der allgemeinen Formel I, in denen $R^1$ $NH_2$ bedeutet, können die Hydroxylamine der allgemeinen Formel VII nach vorheriger Isolierung in an sich bekannter Weise mit 1,1 bis 2 Mol Trimethylsilylisocyanat in einem inerten Lösungsmittel oder Lösungsmittelgemisch, wie cyclischen Ethern, beispielsweise Tetrahydrofuran und/oder 1,4-Dioxan, bei Temperaturen zwischen 20° C und der Siedetemperatur des verwendeten Lösungsmittels umgesetzt und anschließend die intermediär gebildeten Additionsprodukte mit beispielsweise gesättigter Ammonium- oder Natriumchloridlösung hydrolysiert werden. Eine weitere Möglichkeit zur Herstellung der erfindungsgemäßen Verbindungen besteht darin, die Hydroxylamine der allgemeinen Formel VII, gegebenenfalls ohne vorherige Isolierung, mit Kalium- oder Natriumcyanat in saurer Lösung umzusetzen. Darüber hinaus können die Hydroxylamine der allgemeinen Formel VII, gegebenenfalls ohne vorherige Isolierung mit Phosgen in Gegenwart eines säurebindenden Mittels, beispielsweise Natrium- oder Kaliumcarbonat, gefolgt von einer Behandlung mit Ammoniak oder einer Ammoniak liefernden Verbindung, beispielsweise Ammoniumcarbonat, in Oxyalkine der allgemeinen Formel I überführt werden.

Oxyalkine der allgemeinen Formel I, in denen der Rest $R^1$ $CH_3$ bedeutet, können aus den Hydroxylaminen der allgemeinen Formel VII erhalten werden, indem die Hydroxylamine gegebenenfalls ohne vorherige Isolierung mit 2,2 bis 10 Mol eines Acetylierungsmittels, vorzugsweise mit Essigsäureanhydrid, Essigsäurechlorid und/oder Essigsäureethylester, gegebenenfalls unter Verwendung einer Base, wie Pyridin oder Triethylamin, in Gegenwart eines Lösungsmittels, wie Toluol umgesetzt werden. Aus den erhaltenen acetylierten Verbindungen sind durch selektive basische Hydrolyse oder Alkoholyse der O-Acetylgruppe am Stickstoffatom und gegebenenfalls durch Umwandlung der propargylischen $OCOCH_3$-Gruppe in die OH-Gruppe in einem gegebenenfalls wasserhaltigen Lösungsmittel oder Lösungsmittelgemisch, beispielsweise Methanol, Ethanol und/oder Tetrahydrofuran bei Temperaturen zwischen 20° und 60° C die Oxyalkine der allgemeinen Formel I zugänglich. Zur Solvolyse wird als Base Lithium-, Natrium- oder Kaliumhydroxid, Natrium- oder Kaliumcarbonat oder Natriumhydrogencarbonat, gegebenenfalls in Form einer wäßrigen Lösung, verwendet. (J. B. Summers et al., J. Med. Chem. 31, 1960 (1988)).

Oxyalkine der allgemeinen Formel I, die als Racemate oder Diastereomerengemische vorliegen, lassen sich beispielsweise mittels chromatographischer Verfahren gegebenenfalls unter Verwendung chiraler Hilfsstoffe, wie Tribenzoylcellulose, in Enantiomere bzw. Diastereomere überführen.

## Beispiele

Alle Temperaturangaben sind unkorrigiert.

Die $^1$H-NMR-Spektren wurden in deuteriertem Dimethylsulfoxid bei 300 MHz mit dem Gerät AC 300 der Firma Bruker gemessen. Die chemische Verschiebung der spektroskopischen Resonanzdaten ist in ppm angegeben.

Als stationäre Phase für die säulenchromatographischen Trennungen wurde Kieselgel 60 mit einer Korngröße von 0,040 bis 0,063 mm der Fa. E. Merck, Darmstadt, eingesetzt.

Die Mischungsverhältnisse der Laufmittel für die chromatographischen Trennungen sind in Volumen/Volumen angegeben.

Die Mischungsverhältnisse der Lösungsmittelgemische sind in Volumen/Volumen angegeben.

### Beispiel 1

N-Hydroxy-N-[4-[1-hydroxy-3-phenyl-prop-2-in-1-yl]benzyl] acetamid

178,8 ml n-Butyllithium (1,6 molare Lösung in n-Hexan) wurden bei -70° C in einer Stickstoffatmosphäre zu 31,6 ml Phenylacetylen in 600 ml absolutem Tetrahydrofuran getropft. Nach einstündigem Rühren bei -70° C unter Ausschluß von Feuchtigkeit wurde das Gemisch bei -70° C mit 57,4 ml Terephthalaldehydmonodiethylacetal in 600 ml absolutem Tetrahydrofuran versetzt und eine weitere Stunde bei dieser Temperatur gerührt. Anschließend wurde das Reaktionsgemisch zu 500 ml gesättigter Kochsalzlösung gegeben und dreimal mit je 300 ml Essigsäureethylester ausgeschüttelt. Nach Trocknung über Magnesiumsulfat und Abtrennung des Trockenmittels wurde Essigsäureethylester bei 40° C und 2,7 • $10^3$ Pa abdestilliert. Man erhielt 89 g 1-(4-Diethoxymethylphenyl)-3-phenyl-prop-2-in-1-ol als gelbes Öl.

185 g 1-(4-Diethoxymethylphenyl)-3-phenyl-prop-2-in-1-ol, gelöst in 1,8 l Tetrahydrofuran/Wasser 9 : 1, wurden mit 30 g p-Toluolsulfonsäure, Pyridiniumsalz versetzt und 15 Stunden bei 20° C (Raumtemperatur) gerührt. Nach Zugabe von 500 ml gesättigter Natriumhydrogencarbonatlösung wurde die organische Phase abgetrennt und die wäßrige Phase zweimal mit je 200 ml Essigsäureethylester gewaschen. Die vereinigten organischen Phasen wurden über Magnesiumsulfat getrocknet. Nach Abtrennen des Trockenmittels wurde Essigsäureethylester bei 40° C und 2,7 • $10^3$ Pa abdestilliert. Es wurden 131 g 1-(4-Formylphenyl)-3-phenyl-prop-2-in-1-ol als gelbes Öl erhalten, die anschließend zusammen mit 57,9 g Hydroxylammoniumchlorid und 44 g Natriumcarbonat 15 Stunden bei 20° C in 1,1 l Tetrahydrofuran/Wasser 4 : 1 gerührt wurden. Danach wurden 500 ml gesättigter Kochsalzlösung zugegeben. Das Reaktionsgemisch wurde dreimal mit je 200 ml Essigsäureethylester ausgeschüttelt, die vereinigten organischen Phasen über Magnesiumsulfat getrocknet und nach Abtrennung des Trockenmittels durch Filtration bei 40° C und 2,7 • $10^3$ Pa eingeengt. Es wurden 153 g des Oxims 1-(4-Hydroxyiminomethylphenyl)-3-phenyl-prop-2-in-1-ol, syn/anti-Gemisch als farbloser Feststoff erhalten.

10 g des erhaltenen Oxims wurden in 140 ml Tetrahydrofuran gelöst und bei 0° C mit 8 ml Boran-Pyridin-Komplex versetzt. Nach 15 minütigem Rühren wurden bei 0° C 20 ml konzentrierte Salzsäure zugegeben. Nach einstündigem Rühren bei 0° C wurde das Gemisch unter Rühren mit 100 ml Wasser und mit 12,8 g Natriumcarbonat versetzt, wobei der pH-Wert der wäßrigen Phase auf 8,5 anstieg. Nach Abtrennung der organischen Phase wurde die wäßrige Phase mit Essigsäureethylester gewaschen. Die vereinigten organischen Phasen wurden anschließend über Magnesiumsulfat getrocknet. Nach Abtrennung des Trockenmittels wurde Essigsäureethylester bei 40° C und 2,7 • $10^3$ Pa abdestilliert. Es wurden 10,6 g des Hydroxylamins 1-(4-Hydroxyaminomethylphenyl)-3-phenyl-prop-2-in-1-ol als gelbes Öl erhalten.

Zu einer Lösung von 118 g des gemäß der vorhergehenden Stufe hergestellten Hydroxylamins in 850 ml Toluol wurden bei 5 °C 250 ml Essigsäureanhydrid und 200 ml Pyridin getropft. Nach 15-minütigem Rühren bei 5° C und 15-stündigem Rühren bei 20° C wurde das Reaktionsgemisch unter Kühlung zu 150 ml konzentrierter Salzsäure in 3 l Wasser gegeben. Nach Abtrennung der organischen Phase wurde die wäßrige Phase zweimal mit je 500 ml Essigsäureethylester ausgeschüttelt. Die vereinigten organischen Phasen wurden mit 250 ml gesättigter Kochsalzlösung gewaschen und über Magnesiumsulfat getrocknet. Nach Abtrennung des Trockenmittels wurde Essigsäureethylester bei 40° C und 2,7 • $10^3$ Pa abdestilliert. Es wurden 157 g N-Acetoxy-N-[4-[1-acetoxy-3-phenyl-prop-2-in-1-yl]benzyl]acetamid als gelbes Öl erhalten.

140 g des in der vorhergehenden Stufe hergestellten acetylierten Acetamids, gelöst in 1 l Tetrahydrofuran, wurden bei 20° C mit 1 l 2molarer methanolischer Lithiumhydroxidlösung versetzt und 15 Stunden gerührt. Anschließend wurde das Reaktionsgemisch zu 1 l Wasser gegeben und durch Zugabe von 500 ml 10 gew.-%iger Salzsäure auf einen pH-Wert von 5,5 eingestellt. Nach Abtrennung der organischen Phase wurde die wäßrige Phase zweimal mit je 300 ml Essigsäureethylester ausgeschüttelt. Die vereinigten organischen Phasen wurden mit 200 ml gesättigter Kochsalzlösung gewaschen und über Magnesiumsulfat getrocknet. Nach Abtrennung des Trockenmittels wurde Essigsäureethylester bei 40° C und 2,7 • $10^3$ Pa abdestilliert. Es wurden 120 g eines öligen Rückstandes erhalten, der in 1 l Essigsäureethylester gelöst wurde. Anschließend wurde diese Lösung mit 1 l Hexan versetzt, 30 Minuten gerührt und filtriert. Aus dem Filtrat wurden nach Zugabe von 1 l Hexan 56 g farbloses kristallines racemisches N-Hydroxy-N-[4-[1-hydroxy-3-phenyl-prop-2-in-1-yl]benzyl]acetamid mit einem Schmelzpunkt von 121° bis 123° C erhalten.
$^1$H-NMR:
2,04 (s, 3H); 4,76 (s, 2H); 5,31 (s, 1H); 5,63 (d, 3Hz, 1H); 7,30 - 7,44 (m, 7H); 7,57 (d, 8Hz, 2H); 9,43 (s, 1H)

Beispiel 2

(+)-N-Hydroxy-N-[4-[1-hydroxy-3-phenyl-prop-2-in-1-yl] benzyl]acetamid

Das nach Beispiel 1 erhaltene racemische N-Hydroxy-N-[4-[1-hydroxy-3-phenyl-prop-2-in-1-yl]benzyl]acetamid wurde an Tribenzoylcellulose (Riedl de Haen; 10 - 20 µm) mit Methanol/Wasser 90 : 10 chromatographiert.
$[\alpha]^{21}_{D}$:+1,80° (c = 0,06 Mol/l Methanol)
Schmelzpunkt: 112° bis 115° C

Beispiel 3

(-)-N-Hydroxy-N-[4-[1-hydroxy-3-phenyl-prop-2-in-1-yl]benzyl] acetamid

Das nach Beispiel 1 erhaltene racemische N-Hydroxy-N-[4-[1-hydroxy-3-phenyl-prop-2-in-1-yl]benzyl]acetamid wurde unter den in Beispiel 2 angegebenen Bedingungen chromatographiert.

$[\alpha]_D^{21}$: -1,90° (c = 0,05 Mol/l Methanol)
Schmelzpunkt: 117° bis 119° C

Beispiel 4

N-Hydroxy-N-[4-[1-acetoxy-3-phenyl-prop-2-in-1-yl]benzyl] acetamid

38 g N-Acetoxy-N-[4-[1-acetoxy-3-phenyl-prop-2-in-1-yl]benzyl]acetamid, hergestellt nach Beispiel 1, wurden in einem Gemisch aus 180 ml Methanol und 20 ml Wasser gelöst, mit 0,84 g Natriumhydrogencarbonat versetzt und 7 Stunden bei 20° C gerührt. Anschließend wurde das Reaktionsgemisch zu 500 ml gesättigter Kochsalzlösung gegeben und dreimal mit je 50 ml Essigsäureethylester ausgeschüttelt. Die vereinigten organischen Phasen wurden mit 5 ml gesättigter Kochsalzlösung gewaschen, über Magnesiumsulfat getrocknet und nach der Abtrennung des Trockenmittels durch Filtration bei 40° C und 2,7 • $10^3$ Pa Essigsäureethylester abdestilliert. Es wurden 3,3 g Rückstand erhalten, der mit Toluol/Methanol/Essigsäure 4,7 : 0,3 : 0,025 chromatographiert wurde. Es wurden 2,9 g N-Hydroxy-N-[4-[1-acetoxy-3-phenyl-prop-2-in-1-yl]benzyl]acetamid als gelbes Öl erhalten.
$^1$H-NMR:
2,07 (s, 3H); 2,10 (s, 3H); 4,72 (s, 2H); 6,64 (s, 1H); 7,34 - 7,58 (m, 9H); 9,90 (s, 1H)

Beispiel 5

N-Hydroxy-N-[4-[1-ethoxy-3-phenyl-prop-2-in-1-yl]benzyl] acetamid

Zu 5 g des nach Beispiel 1 hergestellten Oxims 1-(4-Hydroxyiminomethylphenyl)-3-phenyl-prop-2-in-1-ol, syn/anti-Gemisch, gelöst in 120 ml Ethanol, wurden bei 0° C 8 ml Boran-Pyridin-Komplex und anschließend 12 ml 20 gew.-%ige ethanolische Salzsäure gegeben. Das Reaktionsgemisch wurde danach 1 Stunde bei 0° C und 2 Stunden bei 20° C gerührt. Anschließend wurde das Reaktionsgemisch zu 1,2 l gesättigter Natriumhydrogencarbonatlösung gegeben und dreimal mit je 400 ml Essigsäureethylester ausgeschüttelt. Die vereinigten organischen Phasen wurden über Magnesiumsulfat getrocknet, filtriert und bei 40° C und 2,7 • $10^3$ Pa Lösungsmittel abdestilliert. Der Rückstand (8,1 g) wurde mit Dichlormethan/Methanol 4,7 : 0,3 chromatographiert. Es wurden 2,3 g 1-(4-Hydroxyaminomethylphenyl)-3-phenyl-1-ethoxy-prop-2-in als farbloses Öl erhalten, das unter den in Beispiel 1 angegebenen Bedingungen in das N-Acetoxy-N-[4-[1-ethoxy-3-phenyl-prop-2-in-1-yl]benzyl]acetamid und anschließend in das gelbe Öl N-Hydroxy-N-[4-[1-ethoxy-3-phenyl-prop-2-in-1-yl]benzyl]acetamid überführt wurde.
$^1$H-NMR:
1,19 (t, 7Hz, 3H); 2,06 (s, 3H); 3,52 - 3,57 (m, 1H); 3,70 - 3,75 (m, 1H); 4,70 (s, 2H); 5,46 (s, 1H); 7,24 - 7,51 (m, 9H); 9,88 (s, 1H)

Beispiel 6

N-Hydroxy-N-[1-[4-(1-hydroxy-3-phenyl-prop-2-in-1-yl)phenyl]ethyl]acetamid

10 g des nach Beispiel 1 hergestellten 1-(4-Formylphenyl)-3-phenyl-prop-2-in-1-ols, gelöst in 200 ml absolutem Tetrahydrofuran, wurden bei 20° C mit 8,7 ml Trimethylchlorsilan und 9,4 ml Triethylamin versetzt und 15 Minuten gerührt. Anschließend wurde das Reaktionsgemisch zu 100 ml gesättigter Natriumhydrogencarbonatlösung und 200 ml gesättigter Kochsalzlösung gegeben und dreimal mit je 50 ml Essigsäureethylester ausgeschüttelt. Die vereinigten organischen Phasen wurden mit 20 ml gesättigter Natriumhydrogencarbonatlösung gewaschen, über Kaliumcarbonat getrocknet, filtriert und bei 40° C und 2,7 • $10^3$ Pa Essigsäureethylester abdestilliert. Der ölige Rückstand wurde mit Hexan/Essigsäureethylester 4,5 : 0,5 chromatographiert. Man erhielt 12 g 1-(4-Formylphenyl)-3-phenyl-prop-2-in-1-trimethylsilylether als gelbes Öl.

5,75 g dieses Silylethers, gelöst in 20 ml absolutem Diethylether, wurden bei 20° C zu einer aus 450 mg Magnesiumspänen und 2,6 g Methyljodid in 90 ml absolutem Diethylether hergestellten Lösung von Methylmagnesiumjodid getropft. Nach 30-minütigem Rühren bei 20° C wurde das Reaktionsgemisch mit 100 ml gesättigter Kochsalzlösung und 10 ml 0,1 molarer Natriumhydroxidlösung versetzt und über Filtererde filtriert. Der feste Rückstand wurde mit 50 ml Diethylether gewaschen und die Waschflüssigkeit mit dem Filtrat vereinigt. Nach Abtrennung der organischen Phase wurde die wäßrige Phase mit Diethylether gewaschen. Die vereinigten organischen Phasen wurden über Kaliumcarbonat getrocknet und nach Abtrennung des Trockenmittels durch Filtration Diethylether bei 40° C und 2,7 • $10^3$ Pa abdestilliert. Der erhaltene ölige Rückstand (5,53 g) wurde mit Hexan/Essigsäureethylester 4 : 1 chromatographiert. Es wurden 2,48 g 1-[4-[1-Hydroxyethyl]phenyl]-3-phenyl-prop-2-in-1-trimethylsilylether als gelbes Öl erhalten, die, in 15,5 ml Dimethylsulfoxid gelöst, bei 20° C mit 13,2 ml Triethylamin und 3,72 g Schwefeltrioxid/Pyridinkomplex versetzt wurden. Nach 45-minütigem Rühren bei 20° C wurde das Reaktionsgemisch unter Kühlung mit 90 ml 1molarer Salzsäure

und 90 ml gesättigter Kochsalzlösung versetzt, 5 Minuten gerührt und anschließend dreimal mit je 50 ml Essigsäureethylester ausgeschüttelt. Die vereinigten organischen Phasen wurden über Magnesiumsulfat getrocknet. Nach Abtrennung des Trockenmittels durch Filtration wurde Essigsäureethylester bei 40° C und 2,7 • $10^3$ Pa abdestilliert. Es wurden 2,62 g eines gelben Öls erhalten, das in 10 ml Essigsäureethylester gelöst wurde und nach Zugabe von 5 ml 1 molarer Salzsäure 15 Stunden bei 20° C gerührt wurde. Anschließend wurde die organische Phase abgetrennt und die wäßrige Phase mit Essigsäureethylester gewaschen. Die vereinigten organischen Phasen wurden über Magnesiumsulfat getrocknet und nach Abtrennung des Trockenmittels Essigsäureethylester bei 40° C und 2,7 • $10^3$ Pa abdestilliert. Der ölige Rückstand (2,42 g) wurde mit Hexan/Essigsäureethylester 3,5 : 1,5 chromatographiert. Es wurden 550 mg 1-[4-[1-Oxoethyl]phenyl]-3-phenyl-prop-2-in-1-ol als gelbes Öl erhalten, das unter den in Beispiel 1 angegebenen Bedingungen nacheinander in das Oxim, Hydroxylamin, acetylierte Acetamid und zuletzt in N-Hydroxy-N-[1-[4-(1-hydroxy-3-phenyl-prop-2-in-1-yl)phenyl]ethyl]acetamid, Diastereomerengemisch überführt wurde.
$^1$H-NMR:
1,48 (d, 7Hz, 3H); 2,04 (s, 3H); 5,61 (s, 1H); 5,65 - 5,68 (m, 1H); 7,35 - 7,54 (m, 9H)

Beispiel 7

N-Hydroxy-N-[1-[4-(1-acetoxy-3-phenyl-prop-2-in-1-yl)phenyl]ethyl]acetamid

Das nach Beispiel 6 hergestellte acetylierte Acetamid N-Acetoxy-N-[1-[4-(1-acetoxy-3-phenyl-prop-2-in-1-yl) phenyl]ethyl]acetamid wurde unter den in Beispiel 4 angegebenen Bedingungen in das Diastereomerengemisch N-Hydroxy-N-[1-[4-(1-acetoxy-3-phenyl-prop-2-in-1-yl)phenyl]ethyl]acetamid überführt.
$^1$H-NMR:
1,47 (d, 7Hz, 3H); 2,03 (s, 3H); 2,10 (s, 3H); 5,63 - 5,66 (m, 1H); 6,64 (s, 1H); 7,45 - 7,56 (m, 9H); 9,58 (s, 1H)

Beispiel 8

N-Hydroxy-N-[4-[1-hydroxy-3-phenyl-prop-2-in-1-yl]benzyl] harnstoff

11,4 g des nach Beispiel 1 hergestellten Hydroxylamins 1-(4-Hydroxyaminomethylphenyl)-3-phenyl-prop-2-in-1-ol wurden in 200 ml Tetrahydrofuran gelöst, mit 7,5 ml Trimethylsilylisocyanat versetzt und eine Stunde unter Rückfluß erhitzt. Nach Abkühlung auf 20° C wurde das Reaktionsgemisch zu 1 l gesättigter Kochsalzlösung gegeben und dreimal mit je 300 ml Essigsäureethylester ausgeschüttelt. Die vereinigten organischen Phasen wurden über Magnesiumsulfat getrocknet, filtriert und bei 40° C und 2,7 • $10^3$ Pa Essigsäureethylester abdestilliert. Der Rückstand (10,4 g) wurde mit Hexan/Isopropanol/Essigsäure 4 :1 : 0,01 chromatographiert und anschließend aus Hexan/ Aceton umkristallisiert. Es wurden 1,5 g N-Hydroxy-N-[4-[1-hydroxy-3-phenyl-prop-2-in-1-yl]benzyl]harnstoff mit einem Zersetzungspunkt bei 145° bis 146° C erhalten.
$^1$H-NMR:
4,56 (s, 2H); 5,59 (d, 6Hz, 1H); 6,14 (d, 6Hz, 1H); 6,37 (s, 2H); 7,30 - 7,51 (m, 9H); 9,39 (s, 1H)

Beispiel 9

N-Hydroxy-N-[4-[1-hydroxy-3-(2-chlorphenyl)prop-2-in-1-yl] benzyl]acetamid

Die Herstellung erfolgte analog Beispiel 1. Anstelle von Phenylacetylen wurde 2-Chlorphenylacetylen eingesetzt.
$^1$H-NMR:
2,05 (s, 3H); 4,69 (s, 2H); 5,65 (s, 1H);6,23 (s, 1H); 7,11 - 7,58 (m, 8H); 9,96 (s, 1H)

Beispiel 10

N-Hydroxy-N-[4-[1-hydroxy-3-(3-chlorphenyl)prop-2-in-1-yl] benzyl]acetamid

Die Herstellung erfolgte analog Beispiel 1. Anstelle von Phenylacetylen wurde 3-Chlorphenylacetylen eingesetzt.
$^1$H-NMR:
2,05 (s, 3H); 4,69 (s, 2H); 5,60 (d, 6Hz, 1H); 6,19 (d, 6Hz, 1H); 7,29 (d, 8Hz, 2H); 7,39 - 7,52 (m, 6H); 9,87 (s, 1H)

Beispiel 11

N-Hydroxy-N-[4-[1-hydroxy-3-(4-chlorphenyl)prop-2-in-1-yl] benzyl]acetamid

Die Herstellung erfolgte analog Beispiel 1. Anstelle von Phenylacetylen wurde 4-Chlorphenylacetylen eingesetzt.
$^1$H-NMR:
2,05 (s, 3H); 4,69 (s, 2H); 5,59 (d, 6Hz, 1H); 6,18 (d, 6Hz, 1H); 7,29 (d, 8Hz, 2H); 7,42 (d, 7Hz, 1H); 7,45 (d, 7Hz, 1H); 7,51 (d, 8Hz, 2H); 9,87 (s, 1H)

Beispiel 12

N-Hydroxy-N-[4-[1-hydroxy-3-(4-fluorphenyl)prop-2-in-1-yl] benzyl]acetamid

Die Herstellung erfolgte analog Beispiel 1. Anstelle von Phenylacetylen wurde 4-Fluorphenylacetylen eingesetzt.
$^1$H-NMR:
2,07 (s, 3H); 4,71 (s, 2H); 5,61 (d, 6Hz, 1H); 6,20 (d, 6Hz, 1H); 7,21 (t, 9Hz, 2H); 7,31 (d, 8Hz, 2H); 7,49 - 7,55 (m, 4H); 9,91 (s, 1H)

Beispiel 13

N-Hydroxy-N-[4-[1-hydroxy-3-(3-methoxyphenyl)prop-2-in-1-yl]benzyl]acetamid

Die Herstellung erfolgte analog Beispiel 1. Anstelle von Phenylacetylen wurde 3-Methoxyphenylacetylen eingesetzt.
$^1$H-NMR:
2,05 (s, 3H); 3,76 (s, 3H); 4,69 (s, 2H); 5,59 (d, 6Hz, 1H); 6,15 (d, 6Hz, 1H); 6,93 - 7,03 (m, 3H); 7,25 - 7,30 (m, 3H); 7,51 (d, 8Hz, 2H); 9,88 (s, 1H)

Beispiel 14

N-Hydroxy-N-[4-[1-hydroxy-3-(3,4-dimethoxyphenyl)prop-2-in-1-yl]benzyl]acetamid

Die Herstellung erfolgte analog Beispiel 1. Anstelle von Phenylacetylen wurde 3,4-Dimethoxyphenylacetylen eingesetzt.
$^1$H-NMR:
2,05 (s, 3H); 3,76 (s, 3H); 3,78 (s, 3H); 4,68 (s, 2H); 5,56 (d, 6Hz, 1H); 6,08 (d, 6Hz, 1H); 6,90 - 7,02 (m, 3H); 7,27 (d, 8Hz, 2H); 7,50 (d, 8Hz, 2H); 9,87 (s, 1H)

Beispiel 15

N-Hydroxy-N-[4-[1-hydroxy-3-(1-naphthyl)prop-2-in-1-yl] benzyl]acetamid

Die Herstellung erfolgte analog Beispiel 1. Anstelle von Phenylacetylen wurde 1-Naphthylacetylen eingesetzt.
$^1$H-NMR:
2,06 (s, 3H); 4,72 (s, 2H); 5,77 (d, 6Hz, 1H); 6,31 (d, 6Hz, 1H); 7,33 (d, 8Hz, 2H); 7,50 (t, 8Hz, 1H); 7,55 - 7,71 (m, 5H); 7,94 - 7,98 (m, 2H); 8,27 (d, 8Hz, 1H); 9,89 (s, 1H)

Beispiel 16

N-Hydroxy-N-[4-[1-hydroxy-3-(2-thienyl)prop-2-in-1-yl] benzyl]acetamid

Die Herstellung erfolgte analog Beispiel 1. Anstelle von Phenylacetylen wurde 2-Thienylacetylen eingesetzt.
Schmelzpunkt: 125° bis 126° C
$^1$H-NMR:
2,04 (s, 3H); 4,68 (s, 2H); 5,60 (d, 6Hz, 1H); 6,18 (d, 6Hz, 1H); 7,05 (t, 4Hz, 1H); 7,26 - 7,29 (m, 3H); 7,47 (d, 8Hz, 2H); 7,56 (d, 5Hz, 1H); 9,84 (s, 1H)

Beispiel 17

N-Hydroxy-N-[4-[1-hydroxy-3-(3-thienyl)prop-2-in-1-yl]benzyl]acetamid

Die Herstellung erfolgte analog Beispiel 1. Anstelle von Phenylacetylen wurde 3-Thienylacetylen eingesetzt.
Schmelzpunkt: 126 - 129° C
$^1$H-NMR:
2,05 (s, 3H); 4,68 (s, 2H); 5,56 (s, 1H); 6,13 (s, 1H); 7,15 (d, 5Hz, 1H); 7,28 (d, 8Hz, 2H); 7,49 (d, 8Hz, 2H); 7,50 - 7,58 (m, 1H); 7,74 (d, 2Hz, 1H); 9,89 (s, 1H)

Beispiel 18

N-Hydroxy-N-[4-[1-hydroxy-3-(3-pyridyl)prop-2-in-1-yl]benzyl]acetamid

Die Herstellung erfolgte analog Beispiel 1. Anstelle von Phenylacetylen wurde 3-Pyridylacetylen eingesetzt.
Schmelzpunkt: 145° - 146° C
$^1$H-NMR:
2,04 (s, 3H); 4,68 (s, 2H); 5,62 (d, 6Hz, 1H); 6,21 (d, 6Hz, 1H); 7,28 (d, 8Hz, 2H); 7,38 - 7,43 (m, 1H); 7,51 (d, 8Hz, 2H); 7,86 (dd, 2Hz, 1H); 8,55 (dd, 1Hz, 1H); 8,63 (s, 1H); 9,36 (s, 1H)

Beispiel 19

N-Hydroxy-N-[3-[1-hydroxy-3-phenyl-prop-2-in-1-yl]benzyl]acetamid

Die Herstellung erfolgte analog Beispiel 1. Anstelle von Terephthalaldehydmonodiethylacetal wurde Isophthalaldehydmonodiethylacetal eingesetzt.
Schmelzpunkt: 104° C
$^1$H-NMR:
2,05 (s, 3H); 4,71 (s, 2H); 5,59 (s, 1H); 6,17 (s, 1H); 7,20 - 7,22 (m, 1H); 7,32 - 7,46 (m, 8H); 9,91 (s, 1H)

Beispiel 20

N-Hydroxy-N-[3-[1-hydroxy-3-(3-thienyl)prop-2-in-1-yl]benzyl]acetamid

Die Herstellung erfolgte analog Beispiel 1. Anstelle von Phenylacetylen wurde 3-Thienylacetylen, anstelle von Terephthalaldehydmonodiethylacetal Isophthalaldehydmonodiethylacetal eingesetzt.
Schmelzpunkt: 133° C
$^1$H-NMR:
2,05 (s, 3H); 4,70 (s, 2H); 5,56 (s, 1H); 6,15 (s, 1H); 7,16 (m, 1H); 7,20 (d, 7Hz, 1H); 7,34 (t, 8Hz, 1H); 7,42 - 7,44 (m, 2H); 7,57 - 7,60 (m, 1H); 7,76 (d, 2Hz, 1H); 9,92 (s, 1H)

Beispiel 21

N-Hydroxy-N-[3-[1-hydroxy-3-(3,4-dimethoxyphenyl)prop-2-in-1-yl]benzyl]acetamid

Die Herstellung erfolgte analog Beispiel 1. Anstelle von Phenylacetylen wurde 3,4-Dimethoxyphenylacetylen, anstelle von Terephthalaldehydmonodiethylacetal Isophthalaldehydmonodiethylacetal eingesetzt.
$^1$H-NMR:
2,05 (s, 3H); 3,77 (s, 6H); 4,71 (s, 2H); 5,60 (s, 1H); 6,12 (s, 1H); 6,91 - 7,04 (m, 3H); 7,20 - 7,22 (m, 1H); 7,32 - 7,37 (m, 1H); 7,44 - 7,47 (m, 2H); 9,91 (s, 1H)

Beispiel 22

N-Hydroxy-N-[4-[1-hydroxy-3-(2-chlorphenyl)prop-2-in-1-yl] benzyl]harnstoff

Analog Beispiel 1 wurde, ausgehend von 2-Chlorphenylacetylen, das entsprechende Hydroxylamin hergestellt, das nach Beispiel 8 in den vorgenannten Harnstoff überführt wurde. Schmelzpunkt: 134° -136° C
$^1$H-NMR:
4,56 (s, 2H); 5,65 (d, 6Hz, 1H); 6,22 (d, 6Hz, 1H); 6,38 (s, 2H); 7,31 - 7,42 (m, 4H); 7,52 - 7,58 (m, 4H); 9,38 (s, 1H)

Beispiel 23

N-Hydroxy-N-[4-[1-hydroxy-3-(3-chlorphenyl)prop-2-in-1-yl] benzyl]harnstoff

Die Herstellung erfolgte analog Beispiel 22. Anstelle von 2-Chlorphenylacetylen wurde 3-Chlorphenylacetylen eingesetzt.
Schmelzpunkt: 118° - 119° C
$^1$H-NMR:
4,54 (s, 2H); 5,59 (d, 6Hz, 1H); 6,18 (d, 6Hz, 1H); 6,36 (s, 2H); 7,31 (d, 8Hz, 2H); 7,36 - 7,50 (m, 6H); 9,36 (s, 1H)

Beispiel 24

N-Hydroxy-N-[4-[1-hydroxy-3-(4-chlorphenyl)prop-2-in-1-yl] benzyl]harnstoff

Die Herstellung erfolgte analog Beispiel 22. Als Alkin wurde 4-Chlorphenylacetylen eingesetzt.
Schmelzpunkt: 140° - 144° C
$^1$H-NMR:
4,52 (s, 2H); 5,57 (d, 6Hz, 1H); 6,15 (d, 6Hz, 1H); 6,34 (s, 2H); 7,30 (d, 8Hz, 2H); 7,40 - 7,48 (m, 6H); 9,35 (s, 1H)

Beispiel 25

N-Hydroxy-N-[4-[1-hydroxy-3-(4-fluorphenyl)prop-2-in-1-yl] benzyl]harnstoff

Die Herstellung erfolgte analog Beispiel 22. Als Alkin wurde 4-Fluorphenylacetylen eingesetzt.
Schmelzpunkt: 136° C
$^1$H-NMR:
4,53 (s, 2H); 5,57 (d, 6Hz, 1H); 6,14 (d, 6Hz, 1H); 6,37 (s, 2H); 7,21 (t, 9Hz, 2H); 7,30 (d, 8Hz, 2H); 7,47 - 7,53 (m, 4H); 9,36 (s, 1H)

Der erhaltene racemische Harnstoff wurde durch Chromatographie an Tribenzoylcellulose (Riedl de Haen; 10 - 20 µm) mit Methanol/Wasser 80 : 20 in seine Enantiomeren getrennt. Die Retentionszeiten betrugen 27,4 Minuten und 34,5 Minuten.

Beispiel 26

N-Hydroxy-N-[4-[1-hydroxy-3-(3-methoxyphenyl)prop-2-in-1-yl]benzyl]harnstoff

Die Herstellung erfolgte analog Beispiel 22. Als Alkin wurde 3-Methoxyphenylacetylen eingesetzt.
$^1$H-NMR:
3,66 (s, 3H); 4,47 (s, 2H); 5,42 (s, 2H); 5,55 (s, 1H); 6,79 (dd, 2Hz, 8Hz, 1H); 6,90 (m, 1H); 6,97 (d, 8Hz, 1H); 7,12 (t, 8Hz, 1H); 7,21 (d, 8Hz, 2H); 7,43 (d, 8Hz, 2H)

Beispiel 27

N-Hydroxy-N-[4-[1-hydroxy-3-(2-thienyl)prop-2-in-1-yl]benzyl]harnstoff

Die Herstellung erfolgte analog Beispiel 22. Als Alkin wurde 2-Thienylacetylen eingesetzt.
$^1$H-NMR:
4,54 (s, 2H); 5,61 (d, 6Hz, 1H); 6,19 (d, 6Hz, 1H); 6,36 (s, 2H); 7,06 (dd, 2Hz, 5Hz, 1H); 7,29 - 7,32 (m, 3H); 7,46 (d, 8Hz, 2H); 7,56 - 7,58 (m, 1H); 9,37 (s, 1H)

Beispiel 28

N-Hydroxy-N-[4-[1-hydroxy-3-(3-thienyl)prop-2-in-1-yl]benzyl]harnstoff

Die Herstellung erfolgte analog Beispiel 22. Als Alkin wurde 3-Thienylacetylen eingesetzt.
Schmelzpunkt: 117° -119° C
$^1$H-NMR:
4,53 (s, 2H); 5,55 (d, 6Hz, 1H); 6,10 (d, 6Hz, 1H); 6,35 (s, 2H); 7,14 - 7,16 (m, 1H); 7,30 (d, 8Hz, 2H); 7,47 (d, 8Hz, 2H); 7,55 - 7,57 (m, 1H); 7,74 - 7,75 (m, 1H); 9,36 (s, 1H)

Beispiel 29

N-Hydroxy-N-[3-[1-hydroxy-3-phenyl-prop-2-in-1-yl]benzyl]harnstoff

Analog Beispiel 1 wurde das Hydroxylamin 1-(3-Hydroxyaminomethylphenyl)-3-phenyl-prop-2-in-1-ol hergestellt. Anstelle von Terephthalaldehydmonodiethylacetal wurde Isophthalaldehydmonodiethylacetal eingesetzt. Das erhaltene Hydroxylamin wurde anschließend gemäß Beispiel 8 in den oben genannten Harnstoff überführt.
Schmelzpunkt: 144° -146° C
$^1$H-NMR:
4,56 (s, 2H); 5,59 (d, 6Hz, 1H); 6,16 (d, 6Hz, 1H); 6,35 (s, 2H); 7,23 - 7,49 (m, 9H); 9,38 (s, 1H)

Beispiel 30

N-Hydroxy-N-[3-[1-hydroxy-3-(3-thienyl)prop-2-in-1-yl]benzyl]harnstoff

Die Herstellung erfolgte analog Beispiel 29. Als Alkin wurde 3-Thienylacetylen eingesetzt.
Schmelzpunkt: 151° C
$^1$H-NMR:
4,55 (s, 2H); 5,55 (d, 6Hz, 1H); 6,13 (d, 6Hz, 1H); 6,55 (s, 2H); 7,16 (dd, 1Hz, 5Hz, 1H); 7,22 (d, 8Hz, 1H); 7,32 (t, 8Hz, 1H); 7,41 (d, 8Hz, 1H); 7,46 (s, 1H); 7,66 (m, 1H); 7,76 (m, 1H); 9,37 (s, 1H)

Beispiel 31

N-Hydroxy-N-[3-[1-hydroxy-3-(3,4-dimethoxyphenyl)prop-2-in-1-yl]benzyl]harnstoff

Die Herstellung erfolgte analog Beispiel 29. Als Alkin wurde 3,4-Dimethoxyphenylacetylen eingesetzt.
Schmelzpunkt: 146° C
$^1$H-NMR:
3,76 (s, 3H); 3,77 (s, 3H); 4,55 (s, 2H); 5,55 (d, 6Hz, 1H); 6,09 (d, 6Hz, 1H); 6,35 (s, 2H); 6,91 - 6,97 (m, 2H); 7,02 (dd, 2Hz, 8Hz, 1H); 7,23 (d, 8Hz, 1H); 7,33 (t, 8Hz, 1H); 7,42 (d, 8Hz, 1H); 7,47 (s, 1H); 9,38 (s, 1H)

Beispiel 32

N-Hydroxy-N-[1-[4-(1-hydroxy-3-phenyl-prop-2-in-1-yl)phenyl]ethyl]harnstoff

Das gemäß Beispiel 6 hergestellte Hydroxylamin 1-[4-[1-Hydroxyaminoethyl]phenyl]-3-phenyl-prop-2-in-1-ol wurde analog Beispiel 8 in den oben genannten diastereomeren Harnstoff überführt.
$^1$H-NMR:
1,42 (d, 7Hz, 3H); 3,50 (s, 2H); 5,33 (q, 7Hz, 1H); 5,57 (s, 1H); 6,29 (s, 2H); 7,35 - 7,48 (m, 9H)

Beispiel 33

N-Hydroxy-N-[4-[1-ethoxy-3-(3,4-dimethoxyphenyl)prop-2-in-1-yl]benzyl]acetamid

Die Herstellung erfolgte analog Beispiel 5. Als Alkin wurde 3,4-Dimethoxyphenylacetylen eingesetzt.
$^1$H-NMR:
1,18 (t, 7Hz, 3H); 2,06 (s, 3H); 3,50 - 3,58 (m, 1H); 3,67 - 3,82 (m, 7H); 4,70 (s, 2H); 5,42 (s, 1H); 6,92 (d, 8Hz, 1H); 6,99 (d, 2Hz, 1H); 7,04 (dd, 8Hz, 2Hz, 1H); 7,31 (d, 8Hz, 2H); 7,50 (d, 8Hz, 2H); 9,89 (s, 1H)

Beispiel 34

N-Hydroxy-N-[4-[1-ethoxy-3-(3,4-dimethoxyphenyl)prop-2-in-1-yl]benzyl]harnstoff

Das ausgehend von 3,4-Dimethoxyphenylacetylen analog Beispiel 5 hergestellte Hydroxylamin 1-[4-Hydroxyaminomethylphenyl]-3-[3,4-dimethoxyphenyl]-1-ethoxy-prop-2-in wurde analog Beispiel 8 in den oben genannten Harnstoff überführt.
$^1$H-NMR:
1,18 (t, 7Hz, 3H); 3,74 - 3,85 (m, 8H); 4,54 (s, 2H); 5,41 (s, 1H); 6,35 (s, 2H); 6,92 (d, 8Hz, 1H); 6,99 (d, 2Hz, 1H); 7,04 (dd, 2Hz, 8Hz, 1H); 7,32 (d, 8Hz, 2H); 7,47 (d, 8Hz, 2H); 9,34 (s, 1H)

Beispiel 35

N-Hydroxy-N-[4-[1-ethoxy-3-(2-thienyl)prop-2-in-1-yl]benzyl]harnstoff

Die Herstellung erfolgte analog Beispiel 34. Als Alkin wurde 2-Thienylacetylen eingesetzt.
$^1$H-NMR:
1,20 (t, 7Hz, 3H); 3,52 - 3,60 (m, 1H); 3,66 - 3,74 (m, 1H); 4,58 (s, 2H); 5,50 (s, 1H); 6,38 (s, 2H); 7,10 (dd, 2Hz, 5Hz, 1H); 7,37 - 7,40 (m, 3H); 7,48 (d, 8Hz, 2H); 7,63 - 7,65 (m, 1H); 9,39 (s, 1H)

Beispiel 36

N-Hydroxy-N-[4-[1-hydroxy-3-(3-pyridyl)prop-2-in-1-yl]benzyl]harnstoff

Ausgehend von 3-Pyridylacetylen wurde analog Beispiel 1 das entsprechende Hydroxylamin hergestellt, das gemäß Beispiel 8 in den oben genannten Harnstoff überführt wurde.
Schmelzpunkt: 145° C
$^1$H-NMR:
4,53 (s, 2H); 5,62 (d, 5,8Hz, 1H); 6,21 (d, 5,9Hz, 1H); 6,35 (s, 2H); 7,29 - 7,55 (m, 5H); 7,84 - 7,87 (m, 1H); 8,53 - 8,55 (m, 1H); 8,62 (d, 1,6Hz, 1H); 9,36 (s, 1H)

Beispiel 37

N-Hydroxy-N-[3-[1-hydroxy-3-(3-pyridyl)prop-2-in-1-yl]benzyl]harnstoff

Die Herstellung erfolgte analog Beispiel 36. Anstelle von Terephthalaldehydmonodiethylacetal wurde Isophthalaldehydmonodiethylacetal eingesetzt.
Schmelzpunkt: 153 ° C
$^1$H-NMR:
4,55 (s, 2H); 5,61 (d, 5,6Hz, 1H); 6,23 (d, 7,5Hz, 1H); 6,34 (s, 2H); 7,22 - 7,49 (m, 5H); 7,86 (d, 7,9Hz, 1H); 8,54 (d, 1,2Hz, 1H); 8,34 (s, 1H); 9,37 (s, 1H)

Beispiel 38

N-Hydroxy-N-[4-[1-hydroxy-3-(4-pyridyl)prop-2-in-1-yl]benzyl]harnstoff

Die Herstellung erfolgte analog Beispiel 36. Als Alkin wurde 4-Pyridylacetylen eingesetzt.
$^1$H-NMR:
4,52 (s, 2H); 5,62 (d, 5,7Hz, 1H); 6,23 (d, 5,8Hz, 1H); 6,33 (s, 2H); 7,30 (d, 8,1Hz, 2H); 7,39 (d, 1,5Hz, 2H); 7,48 (d, 8,1Hz, 2H); 8,56 (d, 1,5Hz, 2H); 9,37 (s, 1H)

Beispiel 39

N-Hydroxy-N-[4-[1-hydroxy-3-(2-pyridyl)prop-2-in-1-yl]benzyl]harnstoff

Die Herstellung erfolgte analog Beispiel 36. Als Alkin wurde 2-Pyridylacetylen eingesetzt.
Schmelzpunkt: 140° -142° C
$^1$H-NMR:
4,52 (s, 2H); 5,61 (d, 5,8Hz, 1H); 6,23 (d, 5,9Hz, 1H); 6,34 (s, 2H); 7,29 - 7,38 (m, 3H); 7,47 - 7,51 (m, 3H); 7,76 - 7,81 (m, 1H); 8,54 (d, 4,8Hz, 1H); 9,34 (s, 1H)

Beispiel 40

N-Hydroxy-N-[3-[1-hydroxy-3-(2-pyridyl)prop-2-in-1-yl]benzyl]harnstoff

Die Herstellung erfolgte analog Beispiel 37. Als Alkin wurde 2-Pyridylacetylen eingesetzt.
Zersetzungspunkt: 108° C
$^1$H-NMR:
4,55 (s, 2H); 5,61 (d, 5,8Hz, 1H); 6,26 (d, 5,8Hz, 1H); 6,34 (s, 2H); 7,23 - 7,52 (m, 6H); 7,76 - 7,82 (m, 1H); 8,54 (d, 4,8Hz, 1H); 9,37 (s, 1H)

Beispiel 41

N-Hydroxy-N-[4-[1-hydroxy-3-(2-pyridyl)prop-2-in-1-yl]benzyl]acetamid

Die Herstellung erfolgte analog Beispiel 1. Als Alkin wurde 2-Pyridylacetylen eingesetzt.
$^1$H-NMR:
2,04 (s, 3H); 4,68 (s, 2H); 5,62 (d, 5,8Hz, 1H); 6,23 (d, 5,9Hz, 1H); 7,27 - 7,49 (m, 6H); 7,76 - 7,88 (m, 1H); 8,54 (d, 4,8Hz, 1H); 9,85 (s, 1H)

Biologische Untersuchungen

Bei allen angegebenen $IC_{50}$-Werten handelt es sich um Absolutwerte.
RP-HPLC bedeutet Reversed-Phase-Hochleistungsflüssigkeitschromatographie.
Als stationäre Phase für die dünnschichtchromatographischen Trennungen wurde Silicagel Si 60 der Firma E. Merck, Darmstadt verwendet.
HETE bedeutet Hydroxyeicosatetraensäure.

Hemmung der 5-Lipoxygenase

1. Hemmung der 5-Lipoxygenase in intakten RBL-1-Zellen
Zur Ermittlung der 5-Lipoxygenasehemmung wurden basophile leukämische Leukozyten der Ratte (RBL-1-Zellen) in vitro gezüchtet, vom Nährboden abzentrifugiert, mit 0,05 molarem Kaliumphosphatpuffer (pH-Wert 7,4) gewaschen und anschließend auf eine Zellzahl von 1 x $10^7$ Zellen pro ml eingestellt. Jeweils 1 ml dieser Zellsuspension wurde mit Indomethacin (10 µmol/l), Calciumchlorid (2 mmol/l) sowie entweder mit einer erfindungsgemäßen Verbindung (0,1 bis 100 µmol/l) oder mit Lösungsmittel (Ethanol und/oder Dimethylsulfoxid) bei Raumtemperatur (20° C) 3 Minuten vorinkubiert und anschließend mit radioaktiv markierter Arachidonsäure (20 µmol/l) und Calciumionophor A 23187 (20 µmol/l) 10 Minuten inkubiert. Nach Abstoppen der Reaktion durch Zugabe von 20 µl Eisessig wurden die Reaktionsprodukte mit Essigsäureethylester extrahiert und mit Laufmittelgemisch dünnschichtchromatographisch getrennt (Jakschik et al., Biochem. Biophys. Res. Commun. 102, 624 (1981)).

Die Radioaktivitätsverteilung der verschiedenen Arachidonsäuremetabolite wurde mit Hilfe eines TLC-Linear-Analyzers gemessen. Aus den prozentualen Bildungsanteilen der 5-Lipoxygenaseprodukte bei Abwesenheit einer erfindungsgemäßen Verbindung sowie in Gegenwart verschiedener Konzentrationen an erfindungsgemäßen Verbindungen wurden graphisch aus halblogarithmischen Diagrammen die $IC_{50}$-Werte, d. h. die Konzentrationen, die eine 50 %ige Hemmung der 5-Lipoxygenase bewirken, bestimmt. Die Ergebnisse sind in der nachfolgenden Tabelle zusammengefaßt:

| erfindungsgemäße Verbindung hergestellt nach Beispiel | $IC_{50}$-Wert in µmolar |
|---|---|
| 1 | 5 |
| 2 | 4 |
| 3 | 5 |
| 4 | 4 |
| 5 | 4 |
| 8 | 7 |
| 9 | 4 |
| 10 | 4 |
| 11 | 8 |
| 13 | 3 |
| 15 | 3 |
| 16 | 3 |
| 17 | 5 |
| 19 | 3 |
| 22 | 4 |
| 23 | 4 |
| 24 | 3 |
| 25 | 3 |
| 26 | 4 |
| 27 | 3 |
| 28 | 4 |
| 29 | 4 |
| 33 | 5 |
| 35 | 3 |

2. Hemmung zellfreier 5-Lipoxygenase aus RBL-1-Zellen

Die zellfreie 5-Lipoxygenase wurde im 10.000 x g Überstand von RBL-1-Zellhomogenaten polarographisch gemessen (M. Haurand und L. Flohé, Biol. Chem. Hoppe-Seyler 369, 133 bis 142 (1988)). Nach 5-minütiger Vorinkubation mit Arachidonsäure (75 µmol/l), Adenosintriphosphat (4 mmol/l), reduziertem Glutathion (4 mmol/l) sowie mit einer erfindungsgemäßen Verbindung (0,1 bis 100 µmol/l) oder Lösungsmittel (Ethanol und/oder Dimethylsulfoxid) bei 35° C wurde die 5-Lipoxygenasereaktion durch Zugabe von Calciumchlorid (3 mmol/l) gestartet und simultan aufgezeichnet.

Die 5-Lipoxygenase-Aktivität wurde aus der Differenz der $pO_2$-Abnahme vor und nach Calciumchloridzugabe bestimmt. Bei Lag-Phasen wurde die Anfangsgeschwindigkeit der Reaktion nach der Lag-Phase ermittelt und die Dauer der Lag-Phase als zusätzlicher Parameter bestimmt. Die geprüften Substanzen zeigten im Konzentrationsbereich zwischen 3 und 50 µmol/l Lag-Phasen von etwa 20 bis 60 Sekunden.

Die ermittelten $IC_{50}$-Werte sind in der nachfolgenden Tabelle zusammengefaßt:

Tabelle

| erfindungsgemäße Verbindung hergestellt nach Beispiel | $IC_{50}$-Wert in µmolar |
|---|---|
| 1 | 5 |
| 4 | 9 |
| 5 | 5 |
| 6 | 8 |
| 8 | 8 |
| 13 | 3 |
| 15 | 7 |
| 16 | 6 |
| 20 | 7 |
| 22 | 8 |
| 25 | 3 |
| 33 | 6 |
| 35 | 5 |

3. Hemmung der FMLP/Merthiolat-induzierten 5-Lipoxygenase von menschlichen polymorphkernigen Leukozyten
Human-Granulozyten wurden mittels Dextran-Sedimentation und Percoll-Gradientenzentrifugation isoliert und mit HBSS (Hanks buffered salt solution) auf eine Zellzahl von 1 x $10^7$/ml eingestellt. Die Zellen wurden bei 37° C 2 Minuten mit einer erfindungsgemäßen Verbindung oder als Kontrolle mit Lösungsmittel (Ethanol und/oder Dimethylsulfoxid) sowie weitere 2 Minuten mit Merthiolat (40 µmol/l) vorinkubiert und anschließend 15 Minuten mit FMLP (Formyl-Methionyl-Leucyl-Phenylalanin; $10^{-7}$mol/l) inkubiert. Die Arachidonsäuremetabolite wurden nach Festphasen-Extraktion mittels RP-HPLC analysiert und quantifiziert (M. Haurand und L. Flohé in Biochem. Pharmacol. 38, 2129 bis 2137 (1989)). Zur Auswertung wurden folgende Arachidonsäuremetabolite bestimmt: 5-HETE, $LTB_4$, 6-trans-$LTB_4$-Isomere, 20-OH-$LTB_4$ und 20-COOH-$LTB_4$.

Zur Untersuchung der Reversibilität der Hemmwirkung wurden die Zellen 5 Minuten bei 37° C mit einer erfindungsgemäßen Verbindung oder als Kontrolle mit Lösungsmittel (Ethanol und/oder Dimethylsulfoxid) inkubiert, 5 Minuten bei 300 x g zentrifugiert und in 2 ml HBSS resuspendiert. Anschließend wurden die Zellen 2 Minuten bei 37° C mit Merthiolat (40 µmol/l) vorinkubiert und 15 Minuten mit FMLP ($10^{-7}$ mol/l) inkubiert.

Die in diesem Testmodell ermittelte reversible Hemmwirkung der nach Beispiel 1 hergestellten erfindungsgemäßen Verbindung betrug $IC_{50}$ = 2 µmolar.

4. Hemmung der 5-Lipoxygenase in Rattenvollblut nach oraler Applikation
Zur Charakterisierung der in-vivo-Verfügbarkeit der erfindungsgemäßen Verbindungen als 5-Lipoxygenasehemmer wurde ein von Tateson et al. in Brit. J. Pharmacol. 94, 528 (1988) beschriebenes Testmodell eingesetzt. Hierzu wurde männlichen Ratten (Stamm Wistar) eine erfindungsgemäße Verbindung peroral appliziert. Jeweils eine Stunde nach Applikation einer erfindungsgemäßen Verbindung wurde den Tieren nach letaler $CO_2$-Narkose unter Zusatz von Heparin als Antikoagulanz Vollblut entnommen. Aliquote des Vollblutes wurden im Wasserbad bei 37° C 30 Minuten mit dem Calciumionophor A 23187 in einer Endkonzentration von 15 µg/ml inkubiert. Anschließend wurden die Inkubationsansätze zentrifugiert und in aliquoten Teilen des zellfreien Plasmas die immunreaktiven Leukotrien $B_4$($iLTB_4$)-Gehalte per Radio-Immun-Assay "RIA" (3H-$LTB_4$-RIA, Fa. Amersham) bestimmt. Der $iLTB_4$-Gehalt jeder Probe wurde anhand einer mit verdünntem Rattenplasma erstellten Standardkurve von $LTB_4$-Standardkonzentrationen rechnerisch ermittelt und als ng $iLTB_4$/ml Rattenplasma berechnet. Parallel zu den mit einer erfindungsgemäßen Verbindung behandelten Tieren wurden jeweils mit Vehikellösung peroral behandelte Tiere als Kontrollgruppe mitgeführt. Der Mittelwert aus den $iLTB_4$-Gehalten der Rattenplasmen dieser Kontrollgruppen diente als 100 %-Wert. Die Aktivität der ex-vivo $iLTB_4$-Synthese nach Applikation einer erfindungsgemäßen Verbindung in % wurde durch Division des Mittelwertes der $iLTB_4$-Gehalte in ng $iLTB_4$/ml der mit einer erfindungsgemäßen Verbindung behandelten Gruppe durch den Mittelwert der $iLTB_4$-Gehalte in ng $iLTB_4$/ml der Kontrollgruppe und

anschließende Multiplikation mit dem Faktor 100 berechnet. Die jeweilige Hemmung in % wurde hieraus durch Substrahieren der %-Aktivität von 100 ermittelt. Bei der Applikation einer Dosis von 21,5 mg/kg peroral ergaben sich für die erfindungsgemäßen Verbindungen die in der nachfolgenden Tabelle zusammengestellten Hemmwerte:

Tabelle

| erfindungsgemäße Verbindung hergestellt nach Beispiel | Hemmung in % |
|---|---|
| 1 | 93 |
| 8 | 65 |
| 10 | 75 |
| 11 | 95 (bei 10 mg/kg) |
| 15 | 71 |
| 17 | 77 |
| 22 | 83 (bei 10 mg/kg) |

Hemmung der 12-Lipoxygenase

1. Hemmung der FMLP/Merthiolat-induzierten 12-HETE- und 5S, 12S-DiHETE-Synthese von menschlichen, polymorphkernigen Leukozyten und Thrombozyten

Coinkubationen von menschlichen Granulozyten und Thrombozyten wurden analog der Hemmung der FMLP/Merthiolat-induzierten 5-Lipoxygenase untersucht. In die Auswertung wurden zusätzlich 12-HETE und 5S,12S-DiHETE mit einbezogen. Die Hemmwirkung der nach Beispiel 1 hergestellten erfindungsgemäßen Verbindung betrug $IC_{50}$ = 5 µmolar.

2. Hemmung der 12-Lipoxygenase in aufgebrochenen, humanen Thrombozytenhomogenaten

Human-Thrombozyten wurden auf eine Zellzahl von 3 • $10^9$/ml eingestellt, mit Stickstoff begast und unter Kühlung in Eiswasser mit Ultraschall beschallt. Anschließend wurde das Homogenat bei 10000 x g 15 Minuten zentrifugiert und mit reduziertem Glutathion (3 mmol/l) versetzt. Je 1 ml 10000 x g Überstand wurde entweder mit einer erfindungsgemäßen Verbindung oder mit Ethanol bei 25° C 4 Minuten und anschließend mit 1-$^{14}$C-markierter Arachidonsäure (40 µmol/l) 7,5 Minuten inkubiert. Zusätzlich wurde den Inkubationsansätzen Indomethacin (10 µmol/l) zugesetzt. Die Reaktion wurde durch Zugabe von Essigsäure beendet, die Arachidonsäuremetabolite mit Essigsäureethylester extrahiert und mittels Dünnschichtchromatographie analysiert. Die Auswertung mittels TLC-Linear Analyzer basierte auf der prozentualen Verteilung der $^{14}$C-markierten Fraktionen. Die $IC_{50}$-Werte wurden graphisch aus halblogarithmischen Diagrammen bestimmt.

Die Hemmwirkung der nach Beispiel 1 hergestellten erfindungsgemäßen Verbindung an 12-Lipoxygenase betrug $IC_{50}$ = 1 µmolar.

3. Hemmung der 12-Lipoxygenase in humanem Vollblut

3 ml Aliquote von heparinisiertem, humanem Vollblut wurden 5 Minuten entweder mit einer erfindungsgemäßen Verbindung oder mit Dimethylsulfoxid bei 37° C vorinkubiert. Anschließend wurde die zellgebundene 12-Lipoxygenase durch Zugabe von Calciumionophor A 23187 (15 µg/ml Blut) aktiviert. Nach 30-minütiger Inkubation bei 37° C und anschließender Zentrifugation wurde zellfreies Plasma gewonnen. Durch Zugabe von 2 ml Ethanol pro ml Plasma wurde Plasmaprotein gefällt, das anschließend durch einen Zentrifugationsschritt abgetrennt wurde. Aus dem mit Wasser auf einen Ethanolgehalt von 10 Vol-% verdünnten Überstand wurde mittels Festphasenextraktion an einer Octadecylsäule 12-HETE als 12-Lipoxygenaseprodukt weiter angereichert. Das methanolische Eluat wurde nach Einengung zur Trockne in 40 vol.-%igem Methanol aufgenommen und HPLC-analytisch auf 12-HETE-Gehalt untersucht.

Die $IC_{50}$-Werte der nach den Beispielen 1, 8 und 16 hergestellten erfindungsgemäßen Verbindungen betrugen 2, 4 und 3 µmolar.

Hemmung der Cyclooxygenase

Schafssamenblasenmikrosomen (80 µg Protein pro ml Puffer; 50 mmolarer Kaliumphosphatpuffer , pH 7,4) wurden in 1 ml Aliquoten bei Raumtemperatur (20° C) 15 Minuten mit Arachidonsäure (20 µmol/l; 150000 dpm; 1-$^{14}$C) sowie mit einer erfindungsgemäßen Verbindung (0,1 bis 100 µmol/l) oder Lösungsmittel (Ethanol und/oder Dimethylsulfoxid) inkubiert. Nach Zugabe von Essigsäure und Extraktion mit Essigsäureethylester wurden die Arachidonsäuremetabolite mittels Dünnschichtchromatographie in eine Prostaglandin- und eine Arachidonsäurefraktion getrennt. Die Auswertung mittels TLC-Linear-Analyzer basierte auf der Bestimmung der prozentualen Verteilung der $^{14}C$-markierten Fraktionen. Die $IC_{50}$-Werte der nach den Beispielen 1, 4, 8, 18, 20, 22, 23, 25 (Racemat und Enantiomere), 26, 28 - 31 und 35 hergestellten erfindungsgemäßen Verbindungen betrugen >500 µ-molar, das bedeutet, daß keine Cyclooxygenasehemmung vorlag.

Allergen-induzierte Bronchokonstriktion beim Meerschweinchen (Konzett-Rössler)

Die antiasthmatische Wirkung der erfindungsgemäßen Verbindungen wurde an narkotisierten, curarisierten und passiv beatmeten Meerschweinchen geprüft. Zur Auslösung einer asthmatischen Reaktion wurden die Tiere passiv mit IgE-haltigem anti-Ovalbumin-Serum (0,2 ml i.p.) sensibilisiert. Nach 48 Stunden wurde die anaphylaktische Bronchokonstriktion durch i.v.-Gabe von 0,2 mg/kg Ovalbumin als Allergen ausgelöst. Die sofort danach auftretende Bronchokonstriktion wurde nach einer modifizierten Methode nach Konzett und Rössler (Naunyn-Schmiedeberg's Arch. Exp. Path. Pharm. 195, 71 bis 74 (1940)) anhand des intratrachealen Druckanstiegs über einen Zeitraum von 15 Minuten gemessen. Effekte, die durch Histamin, Serotonin und sympathische Gegensteuerung ausgelöst werden, wurden durch intravenöse Vorbehandlung der Tiere mit 2,15 mg/kg Mepyramin, 46,4 µg/kg Propranolol, 4,64 mg/kg Atropin und 1 mg/kg Methysergid (5 Minuten vor Auslösung der Bronchokonstriktion durch das Allergen Ovalbumin) ausgeschaltet. Die erfindungsgemäßen Verbindungen wurden bei oraler Applikation eine Stunde vor der Allergengabe gegeben. Die gegenüber Kontrollversuchen (ohne Gabe einer erfindungsgemäßen Verbindung) ermittelten Abnahmen der Bronchokonstriktionen bei Applikation einer erfindungsgemäßen Verbindung sind in Form ihrer $ED_{40}$-Werte, d. h. der effektiven Dosen, bei der die Bronchokonstriktion im Mittel um 40 % gehemmt ist, in der nachfolgenden Tabelle zusammengestellt:

Tabelle

| erfindungsgemäße Verbindung hergestellt nach Beispiel | $ED_{40}$-Wert in mg/kg |
|---|---|
| 1 | 17 |
| 3 | 9 |
| 23 | 18 |
| 25 | 10 |
| 26 | 17 |
| 28 | 17 |

**Patentansprüche**

1. Oxyalkine der allgemeinen Formel I

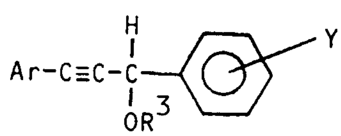

in der der meta- oder para-ständige Substituent Y

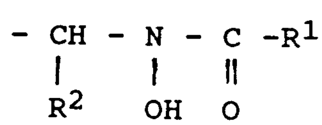

$R^1$ $CH_3$ oder $NH_2$, $R^2$ H oder $CH_3$ und $R^3$ H, $C_{1-3}$-Alkyl oder $COCH_3$ bedeuten und Ar einen aromatischen Rest aus der Gruppe

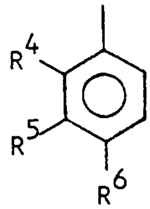 , 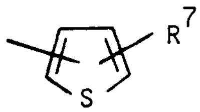 , 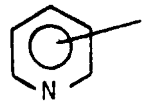 und 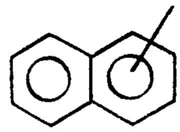

darstellt, mit der Maßgabe, daß die Substituenten $R^4$, $R^5$ und $R^6$ gleich oder verschieden sind und jeweils H, F, Cl, Br, $C_{1-6}$-Alkyl, $CF_3$ oder $C_{1-6}$-Alkoxy bedeuten und $R^7$ H, F, Cl, Br, $C_{1-3}$-Alkyl oder $CF_3$ ist, als Racemate oder Diastereomerengemische oder in optisch aktiver Form.

**2.** Oxyalkine nach Anspruch 1, dadurch gekennzeichnet, daß $R^2$ H bedeutet.

**3.** Oxyalkine nach einem oder beiden der Ansprüche 1 bis 2, dadurch gekennzeichnet, daß $R^3$ H bedeutet.

**4.** Oxyalkine nach einem oder mehreren der Ansprüche 1 bis 3, dadurch gekennzeichnet, daß die Substituenten $R^4$, $R^5$ und $R^6$ gleich oder verschieden sind und jeweils H, F, Cl oder $OCH_3$ bedeuten.

**5.** Oxyalkine nach einem oder mehreren der Ansprüche 1 bis 4, dadurch gekennzeichnet, daß wenigstens einer der Substituenten $R^4$, $R^5$ und $R^6$ H bedeutet.

**6.** Oxyalkine nach einem oder mehreren der Ansprüche 1 bis 5, dadurch gekennzeichnet, daß Ar Phenyl oder 4-Fluorphenyl bedeutet.

**7.** Oxyalkine nach einem oder mehreren der Ansprüche 1 bis 3 dadurch gekennzeichnet, daß $R^7$ H bedeutet.

**8.** Oxyalkine nach einem oder mehreren der Ansprüche 1 bis 7, dadurch gekennzeichnet, daß $R^1$ $CH_3$ bedeutet.

**9.** Oxyalkine nach einem oder mehreren der Ansprüche 1 bis 7, dadurch gekennzeichnet, daß $R^1$ $NH_2$ bedeutet.

**10.** Arzneimittel, dadurch gekennzeichnet, daß es als Wirkstoff wenigstens ein Oxyalkin gemäß den Ansprüchen 1 bis 9 enthält.

**11.** Arzneimittel nach Anspruch 10, dadurch gekennzeichnet, daß es zur oralen Applikation geeignet ist.

**12.** Verfahren zur Herstellung eines Oxyalkins der allgemeinen Formel I

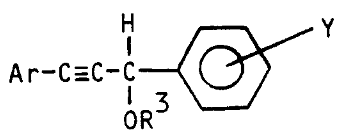

in der der meta- oder para-ständige Substituent Y

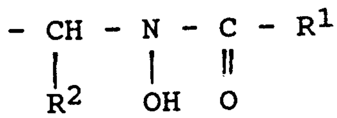

$R^1$ $CH_3$ oder $NH_2$, $R^2$ H oder $CH_3$ und $R^3$ H, $C_{1-3}$-Alkyl oder $COCH_3$ bedeuten und Ar einen aromatischen Rest aus der Gruppe

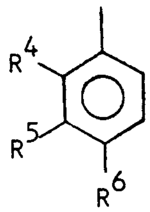, 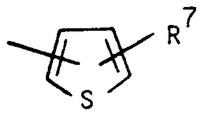, 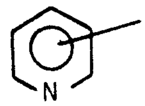 und 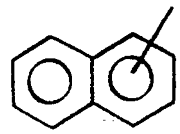

darstellt, mit der Maßgabe, daß die Substituenten $R^4$, $R^5$ und $R^6$ gleich oder verschieden sind und jeweils H, F, Cl, Br, $C_{1-6}$-Alkyl, $CF_3$ oder $C_{1-6}$-Alkoxy bedeuten und $R^7$ H, F, Cl, Br, $C_{1-3}$-Alkyl oder $CF_3$ ist, dadurch gekennzeichnet, daß man ein Alkin der allgemeinen Formel II

$$Ar - C \equiv CH$$

in Gegenwart einer Base mit einem partiell geschützten Terephthal- oder Isophthalaldehyd der allgemeinen Formel III

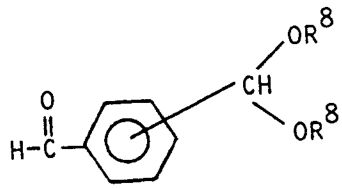

in der $R^8$ $CH_3$ oder $CH_2CH_3$ bedeutet oder die beiden $R^8$-Reste zusammen die Ethylengruppe darstellen, zu einem Acetal der allgemeinen Formel IV

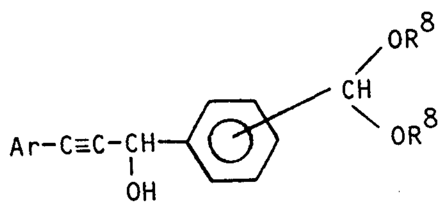

umsetzt, das Acetal mit einer Säure in einem wasserhaltigen Lösungsmittel in einen Hydroxyaldehyd der allgemeinen Formel V

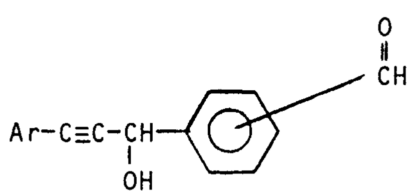

überführt, anschließend aus dem Hydroxyaldehyd ein Oxim der allgemeinen Formel VI

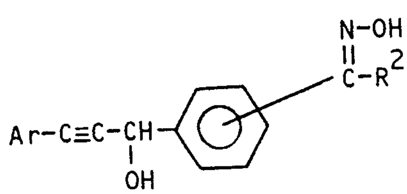

herstellt, indem man entweder
einen Hydroxyaldehyd der allgemeinen Formel V direkt mit Hydroxylamin oder einem seiner Salze in Gegenwart einer Base in ein Oxim der allgemeinen Formel VI überführt
oder
das alkoholische Proton des Hydroxyaldehyds der allgemeinen Formel V durch eine gegenüber Grignardreagenzien inerte, durch Säuren abspaltbare Gruppe ersetzt, anschließend mit einem Methylmagnesiumhalogenid in einem wasserfreien nukleophilen Lösungsmittel umsetzt, den erhaltenen sekundären Alkohol mit einem Oxydationsmittel zu einer Ketoverbindung oxydiert und diese durch Abspaltung der gegenüber Grignardreagenzien inerten Gruppe mit Säuren in eine Ketoverbindung der allgemeinen Formel Va

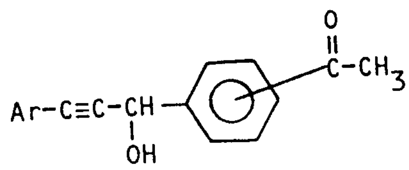

überführt und aus dieser durch Umsetzung mit Hydroxylamin oder einem seiner Salze in Gegenwart einer Base ein Oxim der allgemeinen Formel VI herstellt,
anschließend das erhaltene Oxim mit einem borhaltigen Reduktionsmittel in Gegenwart einer Säure und gegebenenfalls eines $C_{1-3}$-Alkylalkohols zum Hydroxylamin der allgemeinen Formel VII

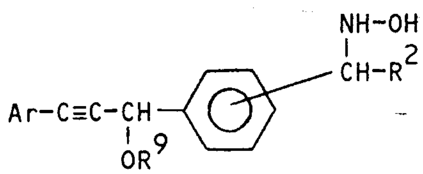

in der $R^9$ H oder $C_{1-3}$-Alkyl bedeutet,
reduziert und das erhaltene Hydroxylamin entweder

A. mit Trimethylsilylisocyanat oder Alkalicyanat und anschließender Hydrolyse oder mit Phosgen und anschließender Aminolyse

oder

B. mit einem Acetylierungsmittel und anschließender selektiver basischer Hydrolyse oder Alkoholyse der O-Acetylgruppe am Stickstoffatom und gegebenenfalls Umwandlung der propargylischen $OCOCH_3$-Gruppe in die OH-Gruppe

in ein Oxyalkin der allgemeinen Formel I überführt.

**13.** Verfahren nach Anspruch 12, dadurch gekennzeichnet, daß man einen Hydroxyaldehyd der allgemeinen Formel V direkt in das Oxim überführt.

**14.** Verfahren nach einem oder beiden der Ansprüche 12 bis 13, dadurch gekennzeichnet, daß man das Oxim in Abwesenheit von $C_{1-3}$-Alkylalkoholen reduziert.

**15.** Verfahren nach einem oder mehreren der Ansprüche 12 bis 14, dadurch gekennzeichnet, daß man ein Oxyalkin der allgemeinen Formel I, in der $R^3$ H bedeutet, herstellt.

## Claims

1. Oxyalkynes of general formula I

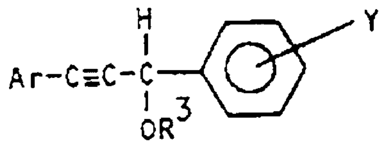

where the meta- or para-substituent Y denotes

- CH - N - C -R¹ ,
|     |    ||
R²   OH  O

$R^1$ denotes $CH_3$ or $NH_2$, $R^2$ denotes H or $CH_3$, and $R^3$ denotes H, $C_1$-$C_3$ alkyl or $COCH_3$, and Ar represents an aromatic radical from the group comprising

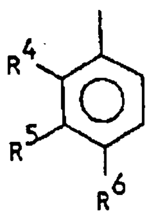 , 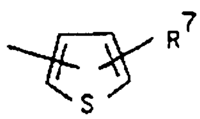 , 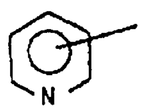 and 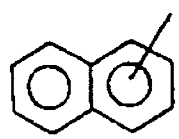

with the proviso that the substituents $R^4$, $R^5$ and $R^6$ are the same or different and each denote H, F, Cl, Br, $C_{1-6}$ alkyl, $CF_3$ or $C_{1-6}$ alkoxy, and $R^7$ is H, F, Cl, Br, $C_{1-3}$ alkyl or $CF_3$, as racemates or mixtures of diastereoisomers or in optically active form.

2. Oxyalkynes according to claim 1, characterised in that $R^2$ denotes H.

3. Oxyalkynes according to one or both of claims 1 to 2, characterised in that $R^3$ denotes H.

4. Oxyalkynes according to one or more of claims 1 to 3, characterised in that the substituents $R^4$, $R^5$ and $R^6$ are the same or different and each denote H, F, Cl or $OCH_3$.

5. Oxyalkynes according to one or more of claims 1 to 4, characterised in that at least one of the substituents $R^4$, $R^5$ and $R^6$ denotes H.

6. Oxyalkynes according to one or more of claims 1 to 5, characterised in that Ar denotes phenyl or 4-fluorophenyl.

7. Oxyalkynes according to one or more of claims 1 to 3, characterised in that $R^7$ denotes H.

8. Oxyalkynes according to one or more of claims 1 to 7, characterised in that $R^1$ denotes $CH_3$.

9. Oxyalkynes according to one or more of claims 1 to 7, characterised in that $R^1$ denotes $NH_2$.

10. A drug, characterised in that it contains at least one oxyalkyne according to claims 1 to 9 as its active ingredient.

11. A drug according to claim 10, characterised in that it is suitable for oral application.

**12.** A method of preparing an oxyalkyne of general formula I

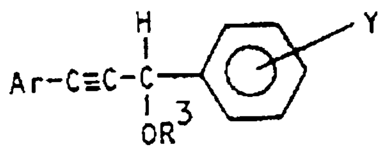

where the meta- or para-substituent Y denotes

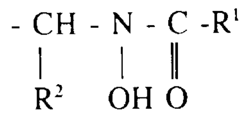

,

$R^1$ denotes $CH_3$ or $NH_2$, $R^2$ denotes H or $CH_3$, and $R^3$ denotes H, $C_1$-$C_3$ alkyl or $COCH_3$, and Ar represents an aromatic radical from the group comprising

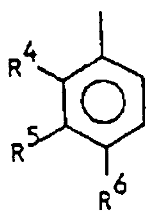 , 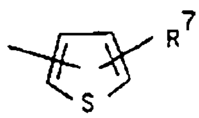 , 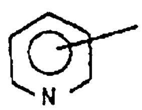 and 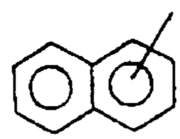

with the proviso that the substituents $R^4$, $R^5$ and $R^6$ are the same or different and each denote H, F, Cl, Br, $C_{1-6}$ alkyl, $CF_3$ or $C_{1-6}$ alkoxy, and $R^7$ is H, F, Cl, Br, $C_{1-3}$ alkyl or $CF_3$, characterised in that an alkyne of general formula II

$$Ar - C \equiv CH$$

is reacted, in the presence of a base, with a partially protected terephthalaldehyde or isophthalaldehyde of general formula III

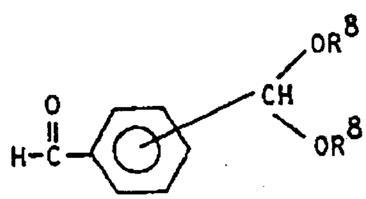

where $R^8$ denotes $CH_3$ or $CH_2CH_3$ or the two $R^8$ radicals together represent an ethylene group, to form an acetal of general formula IV

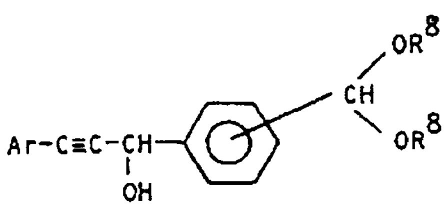

,

the acetal is converted, with an acid in a solvent which contains water, into a hydroxyaldehyde of general formula V

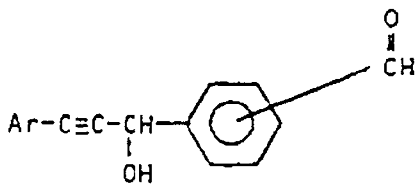

,

and an oxime of general formula VI

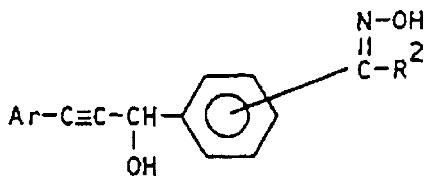

is subsequently prepared from the hydroxyaldehyde, either
by converting a hydroxyaldehyde of general formula V directly into an oxime of general formula VI with hydroxylamine or one of its salts in the presence of a base,
or
by replacing the alcoholic proton of the hydroxyaldehyde of general formula V by a group which is inert to Grignard reagents but which can be split off by acids, subsequent reaction with a methylmagnesium halide in an anhydrous nucleophilic solvent, oxidising the secondary alcohol obtained with an oxidising agent to form a keto compound, and converting the latter, by splitting off with acids the group which is inert to Grignard reagents, into a keto compound of general formula Va

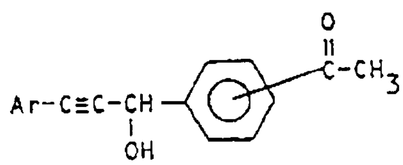

and preparing an oxime of general formula VI from the latter by reaction with hydroxylamine or one of its salts in the presence of a base,
and subsequently reducing the oxime obtained with a reducing agent containing boron in the presence of an acid and optionally of a $C_{1-3}$ alkyl alcohol to form a hydroxylamine of general formula VII

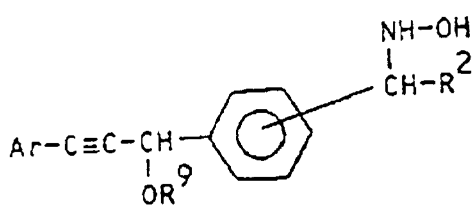

where $R^9$ denotes H or $C_{1-3}$ alkyl,
and converting the hydroxylamine obtained into an oxyalkyne of general formula I either

A. with trimethyl silyl isocyanate or alkali cyanate and subsequent hydrolysis or with phosgene and subsequent aminolysis,

or

B. with an acetylating agent and subsequent selective basic hydrolysis or alcoholysis of the O-acetyl group on the nitrogen atom, optionally with the conversion of the propargylic $OCOCH_3$ group into an OH group.

**13.** A method according to claim 12, characterised in that a hydroxyaldehyde of general formula V is directly converted into the oxime.

**14.** A method according to one or both of claims 12 to 13, characterised in that the oxime is reduced in the absence of $C_{1-3}$ alkyl alcohols.

**15.** A method according to one or more of claims 12 to 14, characterised in that an oxyalkyne of general formula I is prepared, in which $R^3$ denotes H.

**Revendications**

**1.** Oxyalcynes de formule générale I

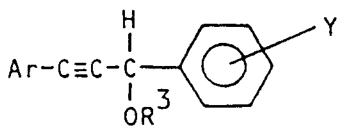

dans laquelle le substituant Y

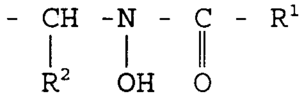

est en position méta ou para,

$R^1$ est un groupe $CH_3$ ou $NH_2$, $R^2$ représente H ou un groupe $CH_3$ et $R^3$ représente H, un groupe alkyle en $C_1$ à $C_3$ ou un groupe $COCH_3$ et Ar est un reste aromatique du groupe

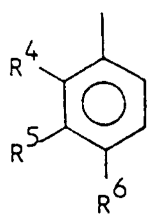 , 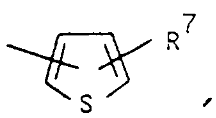 , 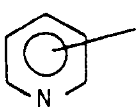 et 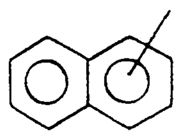

sous réserve que les substituants $R^4$, $R^5$ et $R^6$ soient égaux ou différents et représentent chacun H, F, Cl, Br, un groupe alkyle en $C_1$ à $C_6$, $CF_3$ ou alkoxy en $C_1$ à $C_6$ et que $R^7$ représente H, F, Cl, Br, un groupe alkyle en $C_1$ à $C_3$ ou $CF_3$,

en tant que racémates ou que mélanges de diastéréoisomères ou sous la forme optiquement active.

**2.** Oxyalcynes suivant la revendication 1, caractérisés en ce que $R^2$ représente l'hydrogène.

**3.** Oxyalcynes suivant l'une des revendications 1 et 2 ou les deux, caractérisés en ce que $R^3$ représente l'hydrogène.

**4.** Oxyalcynes suivant l'une ou plusieurs des revendications 1 à 3, caractérisés en ce que les substituants $R^4$, $R^5$ et $R^6$ sont égaux ou différents et représentent chacun H, F, Cl ou un groupe $OCH_3$.

**5.** Oxyalcynes suivant une ou plusieurs des revendications 1 à 4, caractérisés en ce que l'un au moins des substituants $R^4$, $R^5$ et $R^6$ représente de l'hydrogène.

6. Oxyalcynes suivant une ou plusieurs des revendications 1 à 5, caractérisés en ce que Ar est un groupe phényle ou 4-fluorophényle.

7. Oxyalcynes suivant une ou plusieurs des revendications 1 à 3, caractérisés en ce que $R^7$ représente de l'hydrogène.

8. Oxyalcynes suivant une ou plusieurs des revendications 1 à 7, caractérisés en ce que $R^1$ représente le groupe $CH_3$.

9. Oxyalcynes suivant une ou plusieurs des revendications 1 à 7, caractérisés en ce que $R^1$ représente un groupe $NH_2$.

10. Médicament caractérisé en ce qu'il contient comme substance active au moins un oxyalcyne suivant les revendications 1 à 9.

11. Médicament suivant la revendication 10, caractérisé en ce qu'il convient pour l'administration orale.

12. Procédé de production d'un oxyalcyne de formule générale I

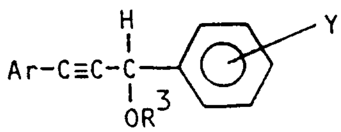

dans laquelle le substituant Y

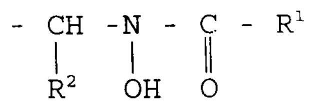

est en position méta ou para,
$R^1$ est un groupe $CH_3$ ou $NH_2$, $R^2$ représente H ou un groupe $CH_3$ et $R^3$ représente H, un groupe alkyle en $C_1$ à $C_3$ ou un groupe $COCH_3$ et Ar est un reste aromatique du groupe

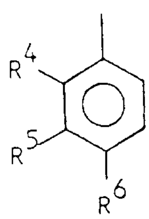 , 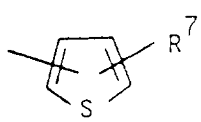 , 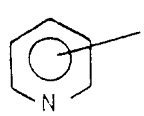 et 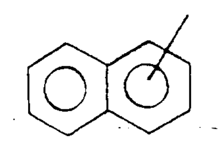

sous réserve que les substituants $R^4$, $R^5$ et $R^6$ soient égaux ou différents et représentent chacun H, F, Cl, Br, un groupe alkyle en $C_1$ à $C_6$, $CF_3$ ou alkoxy en $C_1$ à $C_6$ et que $R^7$ représente H, F, Cl, Br, un groupe alkyle en $C_1$ à $C_3$ ou $CF_3$, caractérisé en ce qu'on fait réagir un alcyne de formule générale II

$$Ar - C \equiv CH$$

en présence d'une base, avec un téréphtalaldéhyde ou un isophtalaldéhyde partiellement protégé de formule générale III

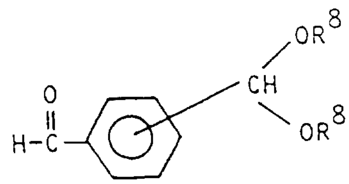

dans laquelle $R^8$ est un groupe $CH_3$ ou $CH_2CH_3$ ou bien les deux restes $R^8$ représentent ensemble le groupe éthylène, pour former un acétal de formule générale IV

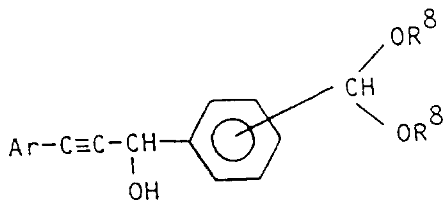

on transforme l'acétal avec un acide dans un solvant contenant de l'eau en un hydroxyaldéhyde de formule générale V

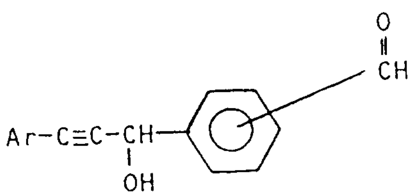

on prépare ensuite à partir de l'hydroxyaldéhyde une oxime de formule générale VI

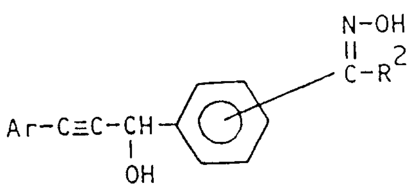

en transformant l'hydroxyaldéhyde de formule générale V directement avec l'hydroxylamine ou avec l'un de ses sels, en présence d'une base, en une oxime de formule générale VI

ou bien

on remplace le proton alcoolique de l'hydroxyaldéhyde de formule générale V par un groupe inerte vis-à-vis de réactifs de Grignard, éliminable par des acides, puis on fait réagir le produit avec un halogénure de méthylmagnésium dans un solvant nucléophile anhydre, on oxyde l'alcool secondaire obtenu avec un agent oxydant en un composé cétonique et on transforme ce dernier par élimination du groupe inerte vis-à-vis de réactifs de Grignard avec des acides en un composé cétonique de formule générale Va

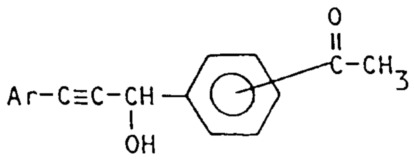

et on prépare à partir de ce composé, par réaction avec l'hydroxylamine ou avec l'un de ses sels en présence d'une base, une oxime de formule générale VI,

puis on réduit l'oxime obtenue avec un agent réducteur contenant du bore, en présence d'un acide et le cas échéant en présence d'un alcanol en $C_1$ à $C_3$, en l'hydroxylamine de formule générale VII

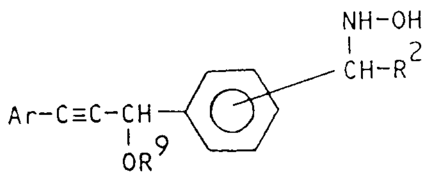

dans laquelle $R^9$ représente H ou un groupe alkyle en $C_1$ à $C_3$ et on transforme en oxyalcyne de formule générale I l'hydroxylamine obtenue

A. avec l'isocyanate de triméthylsilyle ou un cyanate alcalin en effectuant ensuite une hydrolyse, ou avec le phosgène en effectuant ensuite une aminolyse,

ou bien

B. avec un agent d'acétylation en effectuant ensuite une hydrolyse ou une alcoolyse basique sélective du groupe O-acétyle sur l'atome d'azote et le cas échéant la transformation du groupe $OCOCH_3$ propargylique en le groupe OH.

**13.** Procédé suivant la revendication 12, caractérisé en ce qu'on transforme un hydroxyaldéhyde de formule générale V directement en l'oxime.

**14.** Procédé suivant l'une des revendications 12 et 13 ou les deux, caractérisé en ce qu'on réduit l'oxime en l'absence d'alcanols en $C_1$ à $C_3$.

**15.** Procédé suivant une ou plusieurs des revendications 12 à 14, caractérisé en ce qu'on prépare un oxyalcyne de formule générale I dans laquelle $R^3$ désigne l'hydrogène.